(19) Europäisches Patentamt
European Patent Office
Office européen des brevets

(11) **EP 4 410 287 A1**

(12) **EUROPEAN PATENT APPLICATION**

(43) Date of publication:
**07.08.2024 Bulletin 2024/32**

(21) Application number: **23155166.4**

(22) Date of filing: **06.02.2023**

(51) International Patent Classification (IPC):
**A61K 31/436** (2006.01)     **A61K 9/00** (2006.01)
**A61P 37/00** (2006.01)

(52) Cooperative Patent Classification (CPC):
**A61K 31/436; A61K 9/006; A61K 9/7007;**
**A61P 37/00;** A61K 47/38

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Nucleus Medical GmbH**
**82031 Grünwald (DE)**

(72) Inventors:
• **BEIER, Wolfgang.**
  **82031 Grünwald (DE)**
• **HORSTKOTTE, Elke.**
  **82031 Grünwald (DE)**

(74) Representative: **Engelhard, Markus**
**Boehmert & Boehmert**
**Anwaltspartnerschaft mbB**
**Pettenkoferstrasse 22**
**80336 München (DE)**

(54) **A MACROLIDE FOR USE IN A METHOD OF PREVENTING OR TREATING AN IMMUNOLOGICAL DISEASE OR DISORDER**

(57)     The present invention relates to a macrolide for use in a method of preventing or treating an immunological disease or disorder, preferably a transplant rejection, comprising administering said macrolide at least twice daily, wherein said administering at least twice daily comprises administering a first dose at a first time point and a second dose at a second time point, wherein said second time point is at a time point in a range of from about 1 h to about 7 h, preferably of from about 2 h to about 5 h, more preferably of from about 2.5 h to about 3.5 h, even more preferably about 3 h, after said first time point.

EP 4 410 287 A1

**Description**

FIELD OF THE INVENTION

The present invention relates to a macrolide for use in a method of preventing or treating an immunological disease or disorder, preferably a transplant rejection, comprising administering said macrolide at least twice daily, wherein said administering at least twice daily comprises administering a first dose at a first time point and a second dose at a second time point, wherein said second time point is at a time point in a range of from about 1 h to about 7 h, preferably of from about 2 h to about 5 h, more preferably of from about 2.5 h to about 3.5 h, even more preferably about 3 h, after said first time point.

BACKGROUND OF THE INVENTION

Poor water solubility of active agents has been a challenge in the development of pharmaceutical formulations. In fact, the majority of active pharmaceutical ingredients exhibit a low water solubility. To evoke a therapeutic effect, the active agent has to dissolve and reach the bloodstream in sufficient concentration. Accordingly, active agents with low water solubility, such as macrolides, typically have poor bioavailability, i.e. only a small proportion of the administered active agent reaches the bloodstream after ingestion. The low bioavailability of the drugs may relate to incomplete absorption in the gastrointestinal tract and/or metabolism in the gastrointestinal tract. Due to the poor water solubility, oral macrolide formulations, e.g. tacrolimus formulations, have highly variable absorption profiles; for example, the absolute bioavailability of oral tacrolimus formulations is ranging from 5 to 93%. Macrolides such as tacrolimus undergo extensive presystemic metabolism in the GI tract, which limits its oral bioavailability; e.g., in the case of tacrolimus to ~ 25% on average.

Tacrolimus is an exemplary macrolide which is a macrolide lactone with strong immunosuppressive properties that inhibits cellular and humoral immune responses via several mechanisms of action, with the central effect involving inhibition of calcineurin. Tacrolimus is approved as a first-line immunosuppressive agent for the prophylaxis of transplant rejection in liver, kidney, or heart allograft recipients and for treatment of allograft rejection resistant to treatment with other immunosuppressive medicinal products. Since the first approval of Prograf in 1994, oral administered tacrolimus has become a cornerstone in immunosuppression in adult and children undergoing solid organ transplantation (SOT). Approved oral dosage forms of tacrolimus include immediate release capsules (e.g., Prograf® hard capsules), prolonged release capsules (e.g., Advagraf® prolonged-release hard capsules), prolonged release tablets (e.g., Envarsus ® prolonged release tablets) as well as granules (e.g., Modigraf® granules for oral suspension). Oral administration of tacrolimus is now considered the worldwide standard of care in the prophylaxis of rejection after solid organ transplantation. Tacrolimus therapy is a daily, lifelong medication.

Macrolides such as tacrolimus, which has a narrow therapeutic index, exhibit poor oral bioavailability due to such poor solubility, and further show high inter- and intra-individual variability in the pharmacokinetic parameters. Therefore, the doses administered to a patient have to be carefully monitored. Due to the patient-specific dosing of the macrolide tacrolimus (mg/kg body weight/day), the available capsule preparations are often opened, the active ingredient is brought into solution and then applied to the patient; such process being called "drug compounding". Drug compounding does not meet the high quality requirements of a pharmaceutical product, and may even jeopardize drug safety by increasing the risk of incorrect dosing. Furthermore, drug compounding may result in reduced stability of the drug or microbial contamination of the prepared solutions.

Thus, there remains the need for enhanced therapies using macrolides, particularly macrolides for use with increased efficacy and/or enhanced bioavailability. Particularly, treatments that allow to efficiently administer sufficient doses of macrolides to patients are needed. There also remains the need for dosing regimens and/or formulations that allow to effectively deliver macrolides to patients. Particularly, there remains the need for enhanced treatments which allow to achieve sufficient blood concentrations of macrolides. There also remains the need for reducing the variability of the bioavailability of the macrolides, particularly the variability of the macrolide blood levels. Furthermore, there is the need to enhance the bioavailability of macrolides such as tacrolimus. Furthermore, there is the need for enhanced means such as dosing regimens for administering macrolides, e.g. therapeutic systems that allow an efficient administration of active agents such as macrolides. It is also an aim to reduce macrolide doses necessary to achieve a therapeutic effect. Furthermore, there remains the need for efficient methods of preventing or treating immunological diseases or disorders such as transplant rejections.

SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a macrolide for use in a method of preventing or treating an immunological disease or disorder, preferably a transplant rejection, comprising administering said macrolide at least twice daily,

> wherein said administering at least twice daily comprises administering a first dose at a first time point and a second dose at a second time point, wherein said second time point is at a time point in a range of from about 1 h to about 7 h, preferably of from about 2 h to about 5 h, more preferably of from about 2.5 h to about 3.5 h, even more preferably about 3 h, after said first time point;
> wherein, preferably, said macrolide is administered by buccal administration.

In one embodiment, said macrolide is administered in the form of a mucoadhesive layer, preferably mucoadhesive buccal layer; wherein, preferably, said administering comprises attaching said mucoadhesive layer to a body cavity of a patient, preferably to a buccal cavity of a patient and/or to a mucosa of a patient.

In one embodiment, said administering comprises administering each of said first dose and said second dose, preferably each in the form of a mucoadhesive layer, for a duration in a range of from about 1 min to about 7 h, preferably of from about 10 min to about 3 h, more preferably of from about 20 min to about 2 h, even more preferably of from about 30 min to about 90 min, even more preferably of about 60 min.

In one embodiment, said administering comprises administering said first dose for a duration in a range of from about 30 min to about 90 min, preferably for about 60 min; and administering said second dose for a duration in a range of from about 30 min to about 90 min, preferably for about 60 min, at a time point in a range of from about 2.5 h to about 3.5 h, preferably about 3 h, after said first time point.

In one embodiment, said first dose and said second dose, preferably prior to said administration thereof, each comprise said macrolide in an amount in a range of from about 0.001 mg to about 250 mg, preferably in an amount in a range of from about 0.01 mg to about 50 mg, more preferably in a range of from about 0.05 mg to about 25 mg, even more preferably in a range of from about 0.1 mg to about 15 mg, even more preferably in a range of from about 0.5 mg to about 13 mg, optionally in a range of from about 8 mg to about 12 mg; and/or said macrolide is administered by buccal administration, and wherein said administering said macrolide at least twice daily comprises administering a total daily amount of macrolide which is about $\leq 40\%$, preferably about $\leq 30\%$, more preferably about $\leq 25\%$, even more preferably about $\leq 20\%$, of a total daily amount administered by an oral administration.

In one embodiment, said macrolide is present in said mucoadhesive layer, prior to said administration, in an amount in the range of from approximately 0.01 mg to approximately 10 mg, with reference to 1 cm$^2$ of said mucoadhesive layer, preferably in an amount in the range of from approximately 0.1 mg to approximately 5 mg, with reference to 1 cm$^2$ of said mucoadhesive layer, more preferably in the range of from approximately 1.5 mg to approximately 3.5 mg, with reference to 1 cm$^2$ of said mucoadhesive layer, even more preferably in the range of from approximately 1.8 mg to approximately 2.5 mg, with reference to 1 cm$^2$ of said mucoadhesive layer.

In one embodiment, said macrolide is administered in the form of a mucoadhesive layer comprising

- a mucoadhesive polymer, preferably a polymer selected from a) amphiphilic polymers and hydrophilic polymers, b) poly(methacrylates), c) crosslinked polyacrylic acid polymers, d) copolymers of methyl vinyl ether and maleic anhydride, e) polymers comprising polyvinyl acetate and/or polyvinylpyrrolidone, and combinations thereof;

> wherein, preferably,
> the amphiphilic polymers are selected from polyvinyl caprolactam-polyvinyl acetate-polyethylene glycol graft copolymers, polyoxyl castor oils and D-α-tocopherol-polyethylenglycol-succinate (TPGS), and/or
> the poly(methacrylates) are selected from neutral protonizable poly(methacrylates) and cationic poly(methacrylates), preferably selected from copolymers of dialkylaminoethyl methacrylates and methacrylic acid ester(s), and copolymers of trialkylammonioethyl methacrylates and methacrylic acid ester(s), in particular copolymers of dimethylaminoethyl methacrylates and methacrylic acid esters, and copolymers of trimethylammonioethyl methacrylates and methacrylic acid esters;

> and wherein, optionally, the mucoadhesive layer further comprises:
- a cellulose derivative, preferably selected from hydroxypropyl cellulose (HPC), carboxymethyl cellulose (CMC), hydroxypropyl methylcellulose (HPMC), hydroxyethyl cellulose (HEC), methyl cellulose (MC), hydroxyethyl methyl cellulose (HEMC), ethyl cellulose (EC), and a combination thereof;
- a plasticizer; preferably selected from glycerol, polyethylene glycol, e.g. low molecular weight polyethylene glycol such as PEG 200 or PEG 400, propylene glycol, sorbitol, triacetin, and tributylcitrate; more preferably glycerol; and/or
- a colorant, preferably $TiO_2$;

wherein, optionally, said macrolide is present in said layer in the form of micronized macrolide, preferably micronized crystalline macrolide, and/or in a molecularly dissolved non-nanoparticulate form.

In one embodiment, said administering at least twice daily provides a first peak of a macrolide whole blood concentration at a time point in a range of from about 2 h to about 4 h, preferably of from about 2.5 h to about 3.5 h, more preferably about 3 h, after said first time point, and a second peak of said macrolide whole blood concentration at a time point in a range of from about 7 h to about 9 h, preferably of from about 7.5 h to about 8.5 h, more preferably about 8 h, after said first time point;

wherein, optionally, said administering at least twice daily provides said second peak of said macrolide whole blood concentration at a time point in a range of from about 3 h to about 5 h, preferably of from about 3.5 h to about 4.5 h, more preferably about 4 h, after said second time point.

In one embodiment, said administering at least twice daily provides a whole blood concentration of said macrolide in a range of from about 0.01 ng/mL to about 50 ng/mL, preferably in a range of from about 3 ng/mL to about 20 ng/mL, more preferably in a range of from about 5 ng/mL to about 20 ng/mL, for a duration of about 24 hours.

In one embodiment, said administering at least twice daily provides a coefficient of variation below 40%, preferably below 35%, more preferably below 32%, even more preferably 30% or less, such as in a range of from 20% to 30% or in a range of from 25% to 30%.

In one embodiment, said administering at least twice daily provides

- a $t_{max}$ in a range of from about 3 h to about 10 h after said first time point, preferably in a range of from about 7 h to about 9 h after said first time point, more preferably in a range of from about 7.5 h to about 8.5 h after said first time point; and/or
- a $t_{max}$ in a range of from about 1 h to about 6 h after said second time point, preferably in a range of from about 2 h to about 5 h after said second time point, more preferably in a range of from about 3.5 h to about 4.5 h after said second time point, e.g. about 4 h after said second time point.

In one embodiment, said administering at least twice daily provides

- a $C_{max}$ in a range of from about 0.05 ng/mL to about 50 ng/mL, preferably in a range of from about 0.1 ng/mL to about 30 ng/mL, more preferably in a range of from about 0.7 ng/mL to about 20 ng/mL, even more preferably in a range of from about 5.5 ng/mL to about 20 ng/mL; and/or
- a $C_{trough}$ in a range of from about 0.01 ng/mL to about 18 ng/mL, preferably in a range of from about 0.5 ng/mL to about 16 ng/mL, more preferably in a range of from about 3 ng/mL to about 15 ng/mL, such as in a range of from about 3 ng/mL to about 7 ng/mL or in a range of from about 5 ng/mL to about 7 ng/mL.

In one embodiment, said administering at least twice daily is performed for a duration of at least 2 days, preferably at least 1 week, more preferably at least 1 month, even more preferably at least 1 year;

wherein, optionally, if said macrolide is for use in preventing or treating a transplant rejection, said administering at least twice daily is performed for a lifetime of a patient.

In one embodiment, said macrolide is administered to a human patient; wherein, preferably, said patient is not a fasting patient.

In one embodiment, the macrolide is selected from macrolide immune suppressants, macrolide antibiotics, and macrolide antifungals;

wherein, preferably,

- the macrolide immune suppressant is selected from tacrolimus, sirolimus, everolimus, and pimecrolimus;
- the macrolide antibiotic is selected from erythromycin, clarithromycin, azithromycin, roxithromycin, josamycin, spiramycin, telithromycin, tylosin, and fidaxomicin; and/or
- the macrolide antifungal is selected from polyenes, in particular nystatin, natamycin, and amphotericin B,

wherein, more preferably, said macrolide is tacrolimus or sirolimus, even more preferably tacrolimus.

In one embodiment, the mucoadhesive layer is provided in the form of a mucoadhesive film comprising said mucoadhesive layer, preferably in the form of a mucoadhesive buccal film comprising said mucoadhesive layer.

In a further aspect, the present invention relates to a method of preventing and/or treating an immunological disease or disorder, preferably a transplant rejection, comprising administering a macrolide, particularly a therapeutically effective amount of a macrolide, at least twice daily to a patient in need thereof, wherein said administering at least twice daily comprises administering a first dose at a first time point and a second dose at a second time point, wherein said second time point is at a time point in a range of from about 1 h to about 7 h, preferably of from about 2 h to about 5 h, more preferably of from about 2.5 h to about 3.5 h, even more preferably about 3 h, after said first time point; wherein, preferably, said macrolide is administered by buccal administration.

In one embodiment, said macrolide is administered in the form of a mucoadhesive layer, preferably mucoadhesive buccal layer; wherein, preferably, said administering comprises attaching said mucoadhesive layer to a body cavity of a patient, preferably to a buccal cavity of a patient and/or to a mucosa of a patient.

In one embodiment, said administering, said first dose, said second dose, said macrolide, said mucoadhesive layer, and said patient are as defined herein.

In a further aspect, the present invention relates to a use of a macrolide for the manufacture of a medicament for preventing or treating an immunological disease or disorder, preferably a transplant rejection, comprising administering said macrolide at least twice daily,

wherein said administering at least twice daily comprises administering a first dose at a first time point and a second dose at a second time point, wherein said second time point is at a time point in a range of from about 1 h to about 7 h, preferably of from about 2 h to about 5 h, more preferably of from about 2.5 h to about 3.5 h, even more preferably about 3 h, after said first time point; wherein, preferably, said macrolide is administered by buccal administration.

In one embodiment, said macrolide is administered in the form of a mucoadhesive layer, preferably mucoadhesive buccal layer; wherein, preferably, said administering comprises attaching said mucoadhesive layer to a body cavity of a patient, preferably to a buccal cavity of a patient and/or to a mucosa of a patient.

In one embodiment, said administering, said first dose, said second dose, said macrolide, said mucoadhesive layer, and said patient are as defined herein.

DETAILED DESCRIPTION

The present invention aims at, for example, providing an enhanced administration of macrolides, e.g. for preventing and/or treating transplant rejections after transplantations of organs, particularly after solid organ transplantations. The present invention further aims at efficiently administering macrolides. It is also an aim of the invention to provide a macrolide for use in therapy allowing to efficiently achieve therapeutic blood levels of the macrolide. Moreover, it is an aim of the invention to provide a dosing regimen that allows for an early onset of a therapeutic effect. It is further an aim of the invention to reduce side effects. It is also an aim of the invention to reduce doses required to achieve a desired macrolide blood level, such as a blood level within the therapeutic window. The present invention also aims at preventing and treating immunological diseases and conditions such as solid organ transplant rejections. For example, a macrolide for use of the invention may be administered in the form of a mucoadhesive film, e.g. a mucoadhesive, unidirectional, non-dissolvable buccal film. The invention aims at providing efficient therapies, e.g. therapies which are more efficient than standard oral macrolide therapy, such as tacrolimus standard therapy, for example standard therapy involving capsules, tablets, or granules. Although macrolides such as tacrolimus typically have a very low water solubility, the macrolide for use of the invention, e.g. administered in the form of a layer or film, efficiently achieves therapeutic macrolide blood concentrations. Unexpectedly, clinically relevant blood levels of macrolides are achieved with the macrolide for use of the invention.

The term "macrolide", as used herein, relates to compounds of a class of natural products that comprise or consist of a large macrocyclic lactone ring, e.g. a 14-, 15-, or 16-membered lactone ring, to which one or more deoxy sugars, usually cladinose and desosamine, may be attached; e.g. relates to tacrolimus, sirolimus, everolimus, pimecrolimus, erythromycin, clarithromycin, azithromycin, roxithromycin, josamycin, spiramycin, telithromycin, tylosin, and fidaxomicin, nystatin, natamycin, and amphotericin B. In one embodiment, the macrolide is selected from macrolide immune suppressants, macrolide antibiotics, and macrolide antifungals. In one embodiment, the macrolide immune suppressant is selected from tacrolimus, sirolimus, everolimus, and pimecrolimus. In one embodiment, the macrolide antibiotic is selected from erythromycin, clarithromycin, azithromycin, roxithromycin, josamycin, spiramycin, telithromycin, tylosin, and fidaxomicin. In one embodiment, the macrolide antifungal is selected from polyenes, in particular nystatin, natamycin, and amphotericin B. In a preferred embodiment, the macrolide is selected from tacrolimus, sirolimus, everolimus, and pimecrolimus, preferably is selected from tacrolimus and sirolimus, more preferably is tacrolimus. In one embodiment, the terms "active agent", "active ingredient", and "API" relate to the macrolide. In one embodiment, the macrolide is formulated in the form of a mucoadhesive layer.

The term "immunological disease or disorder", as used herein, relates to any immunological disease or disorder known to the person skilled in the art, particularly to diseases, disorders, and/or conditions that can be prevented or treated with a macrolide, such as tacrolimus. For example, an immunological disease or disorder may be selected from transplant rejections, preferably solid organ transplant rejections, such as liver, kidney or heart allograft rejections; autoimmune diseases, such as systemic lupus erythematosus, psoriasis, vitiligo, or lichen ruber planus; infections, such as bacterial infections, e.g. Helicobacter pylori infections or lyme disease; tumors, such as angiofibroma, lymphoma, e.g. T-cell lymphoma, kidney cancer, neuroendocrine tumors, or breast cancer; and inflammatory diseases or conditions, such as atopic eczema, uveitis, dermatitis, Kimura's disease, or inflammations associated with coronary heart disease. For example, an immunological disease or disorder to be prevented or treated with a macrolide, such as tacrolimus, may be selected from transplant rejections, preferably solid organ transplant rejections, such as liver, kidney or heart allograft rejections; autoimmune diseases, such as psoriasis; infections; tumors; and inflammatory diseases, disorders, or conditions, such as atopic eczema, uveitis, or Kimura's disease. In one embodiment, the immunological disease or disorder is selected from transplant rejections, preferably solid organ transplant rejections, such as liver, kidney or heart allograft rejections; autoimmune diseases, such as systemic lupus erythematosus, psoriasis, vitiligo, or lichen ruber planus; infections, such as bacterial infections, e.g. Helicobacter pylori infections or lyme disease; tumors, such as angiofibroma, lymphoma, e.g. T-cell lymphoma, kidney cancer, neuroendocrine tumors, or breast cancer; and inflammatory diseases or conditions, such as atopic eczema, uveitis, dermatitis, Kimura's disease, or inflammations associated with coronary heart disease. In a preferred embodiment, the immunological disease or disorder is a transplant rejection. In one embodiment, the immunological disease or disorder is a transplant rejection, preferably a solid organ transplant rejection, such as a liver, kidney or heart allograft rejection. In one embodiment, the terms "immunological disorder" and "immunological condition" are used interchangeably. In a preferred embodiment, the macrolide is for use in preventing or treating a transplant rejection.

In one embodiment, said administering said macrolide at least twice daily comprises administering said macrolide by mucosal administration, preferably by buccal administration. In one embodiment, said administering at least twice daily comprises administering the first dose and second dose, optionally further dose(s), by mucosal administration, preferably by buccal administration. For example, mucosal administration may involve administration to moist cavities, such as the lining of the mouth or nose. Advantageously, mucosal administration, such as buccal administration, allows for reduced systemic side-effects. Advantageously, the mucosal route, particularly the buccal route, can avoid significant drawbacks present for the oral administration of drugs, such as hepatic first-pass metabolism, slow absorption, and drug degradation within the gastrointestinal tract. For example, buccal administration may be a topical route of administration by which drugs held or applied in the buccal area diffuse through the oral mucosa and may enter directly into the bloodstream. In a preferred embodiment, said mucosal administration, preferably said buccal administration, comprises administering said macrolide in the form of a mucoadhesive layer, particularly in the form of a mucoadhesive layer comprising said macrolide. Advantageously, buccal administration provides a better bioavailability and may provide a more rapid onset of action compared to oral administration because the medication does not pass through the digestive system and thereby avoids first pass metabolism. The inventors have found that, advantageously, when administering two doses by mucosal administration such as buccal administration, particularly in a dosing interval of 7 h or less, preferably 5 h or less, more preferably 4 h or less, even more preferably in a range of from about 2.5 h to about 3.5 h, desired macrolide blood levels may be efficiently achieved and the dose required for desired macrolide blood levels can be greatly reduced, e.g. by about 80%. Advantageously, the buccal administration requires only 20-25% of the dose of a standard oral therapy to achieve clinically relevant blood levels. In a preferred embodiment, said macrolide is administered to the buccal mucosa. From a drug delivery standpoint, the buccal mucosa offers advantages over other epithelia in the oral cavity including a larger surface area and is highly vascularized resulting in a direct access to the systemic circulation via capillaries and venous drainage, bypassing the hepatic first-pass-effect.

The term "at least twice daily", as used herein, relates to the macrolide being administered in the form of at least two doses per day; for example, the macrolide being administered in the form of a first dose and a second dose, and optionally one or more further doses such as a third dose. The number of doses may be adjusted to meet the patient's needs; thus, if necessary, more than two doses per day may be administered, e.g. to a patient having a high body weight. In one embodiment, at least twice daily relates to twice daily, three times daily, or four times daily, preferably to twice daily. In a preferred embodiment, said macrolide is administered twice daily. In a preferred embodiment, said administering comprises administering said macrolides twice daily. Optionally, more than two doses per day are administered to said patient. In one embodiment, said administering at least twice daily comprises administering a first dose at a first time point and a second dose at a second time point, optionally a third dose at a third time point, wherein said second time point is at a time point in a range of from about 1 h to about 7 h, preferably of from about 2 h to about 5 h, more preferably of from about 2.5 h to about 3.5 h, even more preferably about 3 h, after said first time point. In one embodiment, said administering at least twice daily comprises administering a first dose in the form of a mucoadhesive layer and a second dose in the form of a mucoadhesive layer, optionally a third dose in the form of a mucoadhesive layer.

Advantageously, when administering a second dose at a time point in a range of from about 1 h to about 7 h, preferably of from about 2 h to about 5 h, more preferably of from about 2.5 h to about 3.5 h, even more preferably about 3 h, after said first time point, i.e. after said administration of said first dose, exceptionally high blood levels are achieved and the required dose is reduced. Advantageously, the blood level achieved when administering the second dose at the second time point in a range of from about 1 h to about 7 h after said first time point, for example about 3 h after said first time point, is much higher than the expected blood level. Particularly, the blood level achieved with the second dose is higher than twice as much as the blood level achieved with the first dose. Thus, a synergistic effect may be achieved when administering the second dose at a second time point in a range of from about 1 h to about 7 h, preferably of from about 2 h to about 5 h, more preferably of from about 2.5 h to about 3.5 h, even more preferably about 3 h, after said first time point. The inventors have found that, surprisingly, when the second dose is administered within 7 hours, preferably 5 hours, more preferably 4 hours, even more preferably 3 hours, after the administration of said first dose, the dose required to achieve a certain blood level can be greatly reduced compared to when administering a second dose more than 9 h, particularly more than 10 h, e.g. about 12 h, after said first dose. The inventors have found that the highest synergistic effect is achieved when the second dose is administered at a second time point in a range of from about 2.5 h to about 3.5 h, such as about 3 h, after said first time point. Particularly, the inventors have found that, surprisingly, when the second dose is administered at a second time point in a range of from about 2.5 h to about 3.5 h, such as about 3 h, after said first time point, the blood level achieved with the second dose greatly exceeds the blood level achieved with the first dose by a factor of more than two. Furthermore, the inventors have found that, surprisingly, when the second dose is administered at a second time point within 7 hours, particularly in a range of from about 2.5 h to about 3.5 h, such as about 3 h, after said first time point, the dose required to achieve a certain blood level is highly reduced. The inventors have found that these advantages are strongest if the second dose is administered within 3,5h after said first time point, particularly in a range of from about 2.5 h to about 3.5 h after said first time point. In one embodiment, the terms "b.i.d.", "bid", and "twice daily" are used interchangeably. In one embodiment, the first dose is administered in the form of a first mucoadhesive layer and the second dose is administered in the form of a second mucoadhesive layer. Optionally, further dose(s) may be administered in the form of further mucoadhesive layer(s).

The terms "first time point" and "second time point", as used herein, preferably relate to time points when the first dose and the second dose, respectively, are initially administered. For example, the first time point relates to a time point in which a mucoadhesive layer is initially attached to a body cavity, and the second time point relates to a time point in which a second mucoadhesive layer is initially attached to the body cavity. The mucoadhesive layers may then stay attached to the body cavity for a duration of, for example, about 1 min to about 7 h, such as about 60 min. Preferably, the second time point is at a time point in a range of from about 1 h to about 7 h after said first time point, wherein said first time point relates to the initial administration of the first dose and is independent of the duration of the administration of the first dose. For example, the first time point may relate to t=o, i.e. to the time point of administering the first dose, such as the time point of attaching the mucoadhesive layer. The first time point preferably relates to the start of the prevention or treatment. The term "second time point", as used herein, relates to a time point in a range of from about 1 h to about 7 h, preferably of from about 2 h to about 5 h, more preferably of from about 2.5 h to about 3.5 h, even more preferably about 3 h, after said first time point.

The inventors have found that such an asymmetric administration of at least two doses, i.e. administering the second dose within about 7 hours, preferably within about 5 hours, more preferably within about 4 hours, even more preferably within about 3.5 hours, instead of administering the second dose about 10 h to about 12 h after the first dose, allows to achieve higher macrolide blood levels. Advantageously, since desired blood levels are achieved more efficiently, the applied dose can be reduced. Thus, for example, side effects may be reduced by applying lower doses.

In a preferred embodiment, said macrolide is administered by mucosal administration, preferably by buccal administration. The mucoadhesive layer, e.g. a mucoadhesive layer comprised by a mucoadhesive film, may be attached to a body cavity of a patient, such as may be attached in the mouth, nose, or rectum, of a patient. In a preferred embodiment, the mucoadhesive layer is attached to a mucosa of a body cavity of a patient. In one embodiment, said mucoadhesive layer is attached to a buccal cavity, particularly to a mucosa of the buccal cavity. Advantageously, by attaching the mucoadhesive layer to the mucosa, the macrolide is directly released into the mucosa, optionally forming a depot in the mucosa, and the first-pass effect is circumvented. Advantageously, the direct administration of the macrolide to the mucosa allows to achieve desired blood levels by administering doses that are reduced compared to doses for oral administration, e.g. compared to doses administered in the form of tablets or pills. In a preferred embodiment, the body cavity to which the layer is attached is the oral cavity, particularly the buccal cavity. In one embodiment, said administering involves an attachment of a mucoadhesive layer, e.g. in the form of a mucoadhesive film, in a body cavity, preferably the buccal cavity, of a patient, wherein said attachment is such that said mucoadhesive film makes contact with a site of attachment in the body cavity, preferably buccal cavity, particularly a mucosa, via a side of said polymeric matrix layer that has no backing layer attached.

In one embodiment, said administering comprises administering each of said first dose and said second dose, optionally of said further dose(s), for a duration in a range of from about 1 min to about 7 h, preferably of from about 10 min to about 3 h, more preferably of from about 20 min to about 2 h, even more preferably of from about 30 min to about 90 min, even more preferably of about 60 min. For example, the first dose is administered at a first time point, e.g. in the form of a mucoadhesive layer, and then remains at the site of administration, preferably at the mucosa, more preferably at the buccal cavity, for a duration in a range of from about 1 min to about 7 h, preferably of from about 10 min to about 3 h, more preferably of from about 20 min to about 2 h, even more preferably of from about 30 min to about 90 min, even more preferably of about 60 min. For example, the second dose is administered at the second time point, e.g. in the form of a mucoadhesive layer, and then remains at the site of administration, preferably at the mucosa, more preferably at the buccal cavity, for a duration in a range of from about 1 min to about 7 h, preferably of from about 10 min to about 3 h, more preferably of from about 20 min to about 2 h, even more preferably of from about 30 min to about 90 min, even more preferably of about 60 min. Advantageously, by administering said doses, e.g. in the form of mucoadhesive layers, for a duration in a range of from about 1 min to about 7h, such as from about 30 min to about 90 min, the macrolide can be directly released into the mucosa, for example in the buccal cavity, and can efficiently enter the blood system of the patient. After said duration, the administration device, particularly the mucoadhesive layer, may be removed. In one embodiment, an amount of from about 50% to about 95%, preferably of from about 70% to about 93%, of the initial macrolide amount is contained in the mucoadhesive layer(s) at the end of said duration. For example, when removing a first mucoadhesive layer comprising the first dose after said duration, the first mucoadhesive layer comprises about 50% to about 95%, preferably of from about 70% to about 93%, of the initial macrolide amount which was comprised in said layer prior to said administration. For example, when removing a second mucoadhesive layer comprising the second dose after said duration, the second mucoadhesive layer comprises about 50% to about 95%, preferably of from about 70% to about 93%, of the initial macrolide amount which was comprised in said layer prior to said administration.

Particularly, the inventors have found that a highly efficient and synergistic administration of at least two doses can be achieved when said administering comprises administering said first dose for a duration in a range of from about 30 min to about 90 min, preferably for about 60 min; and administering said second dose for a duration in a range of from about 30 min to about 90 min, preferably for about 60 min, at a time point in a range of from about 2.5 h to about 3.5 h, preferably about 3 h, after said first time point. Advantageously, with such an asymmetric administration, very high blood levels may be achieved, particularly even with relatively low doses.

In one embodiment, said first dose and said second dose, optionally said further dose(s), preferably prior to said administration thereof, each comprise said macrolide in an amount in a range of from about 0.001 mg to about 250 mg, preferably in an amount in a range of from about 0.01 mg to about 50 mg, more preferably in a range of from about 0.05 mg to about 25 mg, even more preferably in a range of from about 0.1 mg to about 15 mg, even more preferably in a range of from about 0.5 mg to about 13 mg, optionally in a range of from about 8 mg to about 12 mg. In one embodiment, a total daily dose comprises said macrolide in an amount in a range of from about 0.001 mg to about 500 mg, preferably in an amount in a range of from about 0.01 mg to about 100 mg, more preferably in a range of from about 0.05 mg to about 50 mg, even more preferably in a range of from about 0.1 mg to about 30 mg, even more preferably in a range of from about 0.5 mg to about 26 mg, optionally in a range of from about 8 mg to about 24 mg. The term "total daily dose", as used herein, preferably relates to the total amount of macrolide administered per day, such as the total amount of the first dose and the second dose. In one embodiment, the terms "total daily dose" and "total daily amount" are used interchangeably. If two doses are applied per day, the total daily dose preferably relates to the sum of the first dose and the second dose. If more than two doses are applied per day, the total daily dose preferably relates to the sum of the first dose, the second dose, and the further dose(s). Advantageously, by administering a second dose within 7 hours, preferably within 5 hours, more preferably within 3.5 hours, such as within 3 hours, after said first dose, particularly when administering the doses in the form of mucoadhesive layers, the first dose, the second dose, and/or the total daily dose required to achieve a desired macrolide blood level is/are greatly reduced compared to when the second dose is applied at a time point in a range of from about 10 h to about 14 h, e.g. about 12 h, after said first dose.

In one embodiment, said macrolide is administered by mucosal administration, preferably by buccal administration, and said administering said macrolide at least twice daily comprises administering a total daily amount of macrolide which is about ≤ 40%, preferably about ≤ 30%, more preferably about ≤ 25%, even more preferably about ≤ 20%, of a total daily amount administered by an oral administration. For example, if the recommended total daily oral dose is 100 mg, the macrolide for use of the invention is applied in a total daily amount of ≤ 40mg, preferably about ≤ 30mg, more preferably about ≤ 25mg, even more preferably about ≤ 20mg. The skilled person understands the recommended total daily oral dose in view of the package leaflet of oral formulations, such as Prograf®. In one embodiment, said macrolide is administered by mucosal administration, preferably by buccal administration, and said administering said macrolide at least twice daily comprises administering a total daily amount of macrolide which comprises or consists of about ≤ 40% of a recommended total daily oral dose, preferably about ≤ 30% of a recommended total daily oral dose, more preferably about ≤ 25% of a recommended total daily oral dose, even more preferably about ≤ 20% of a recommended total daily oral dose. The term "recommended total daily oral dose", as used herein, preferably relates to a total daily dose having marketing approval for an oral formulation of a macrolide and/or being listed in a package leaflet of an oral macrolide formulation. When referring to the term "oral" in the context of an oral administration, oral formulation, and/or oral dose, the term preferably relates to an administration/formulation/dose involving swallowing, such as an oral administration comprising swallowing a tablet, capsule, granules, or a pill, so that the macrolide enters the blood system via the gastrointestinal tract; in contrast thereto, a "buccal" administration/formulation/dose does not involve swallowing significant amounts of the macrolide, but instead, the macrolide is administered to the blood system via the mucosa of the cheek. In one embodiment, an oral administration relates to a peroral administration. In one embodiment, an oral dose relates to a peroral dose. Advantageously, in view of the highly efficient administration, the macrolide for use of the invention allows to apply much lower amounts of the macrolide, e.g. only about 20% of the recommended oral dose, and still achieves therapeutic levels. Advantageously, the bioavailability of the macrolide is greatly enhanced when being administered mucosally, particularly buccally, instead of orally, particularly when a second dose is administered within 7 h after administering a first dose. Without wishing to be bound by any theory, the inventors believe that the enhanced bioavailability of the macrolide for use of the invention is provided the first-pass-effect and the pre-systemic degradation after oral administration are not relevant in mucosal, particularly buccal application. Advantageously, particularly when the first and second doses are provided in the form of a mucoadhesive layer, by administering the second dose within 7h, preferably within 5 h, more preferably within 3.5 h, after the first dose, it is possible to reduce the drug load for the patient by approx. 80%. For example, thereby the side effects of macrolide therapy may be reduced.

The inventors have found that the administration of the macrolide is even further enhanced when said macrolide is present in said mucoadhesive layer, prior to said administration, in an amount in the range of from approximately 0.01 mg to approximately 10 mg, with reference to 1 cm$^2$ of said mucoadhesive layer, preferably in an amount in the range of from approximately 0.1 mg to approximately 5 mg, with reference to 1 cm$^2$ of said mucoadhesive layer, more preferably in the range of from approximately 1.5 mg to approximately 3.5 mg, with reference to 1 cm$^2$ of said mucoadhesive layer, even more preferably in the range of from approximately 1.8 mg to approximately 2.5 mg, with reference to 1 cm$^2$ of said mucoadhesive layer. The expressions "prior to said administration" and "prior to said administration thereof, as used herein in the context of amounts or doses of the macrolide, are meant to be understood as relating to the mucoadhesive layer comprising the respective amount or dose after its preparation and before its administration, particularly the initial amount/dose comprised by the mucoadhesive layer; when the mucoadhesive layer is administered, e.g. attached to the buccal cavity, the amount/dose of macrolide is typically reduced over time, since the macrolide is released from the mucoadhesive layer into the buccal cavity.

In a preferred embodiment, said administering at least twice daily provides a first peak of a macrolide whole blood concentration at a time point in a range of from about 2 h to about 4 h, preferably of from about 2.5 h to about 3.5 h, more preferably about 3 h, after said first time point, and a second peak of said macrolide whole blood concentration at a time point in a range of from about 7 h to about 9 h, preferably of from about 7.5 h to about 8.5 h, more preferably about 8 h, after said first time point. In one embodiment, said administering at least twice daily provides said second peak of said macrolide whole blood concentration at a time point in a range of from about 3 h to about 5 h, preferably of from about 3.5 h to about 4.5 h, more preferably about 4 h, after said second time point. Preferably, the second peak is higher than the first peak. Preferably, the second peak is higher than the first peak by a factor of at least two. For example, the second peak is twice as high as the first peak or is higher than twice as high as the first peak. For example, the sum of the heights of the first and second peak may be higher than the twice the height of the first peak. Advantageously, the first dose and the second dose act synergistically when applied within 7h or less, preferably 5h or less, particularly 3.5h or less. The inventors have found that, surprisingly, the second peak is higher than expected since it greatly exceeds the first peak. In one embodiment, the terms "whole blood concentration" and "blood concentration" are used interchangeably. Preferably, when relating to a "whole blood concentration", "blood concentration", or "blood level", the macrolide blood concentration is meant.

In one embodiment, said administering at least twice daily provides a whole blood concentration of said macrolide in a range of from about 0.01 ng/mL to about 50 ng/mL, preferably in a range of from about 3 ng/mL to about 20 ng/mL, more preferably in a range of from about 5 ng/mL to about 20 ng/mL, for a duration of about 24 hours. Advantageously, the macrolide for use of the invention allows to achieve a desired blood concentration, such as a blood concentration in a range of from about 5 ng/mL to about 20 ng/mL. In one embodiment, the therapeutic window is in a range of from about 5 ng/mL to about 20 ng/mL. In a preferred embodiment, the macrolide for use of the invention is administered such that the blood concentration is within the therapeutic window.

The inventors have found that, surprisingly, the coefficient of variation of the macrolide for use of the invention is greatly reduced compared to a standard oral administration of macrolide, such as a Prograf® administration twice daily with a dosing interval of about 10 h to 12 h. Particularly, the coefficient of variation is even further enhanced, i.e. reduced, when the macrolide for use of the invention is administered in the form of mucoadhesive layers. Advantageously, an intra-subject and an inter-subject reduction of the coefficient of variation is observed with the macrolide for use of the invention compared to a standard oral administration of macrolide, such as a Prograf® administration twice daily with a dosing interval of about 10 h to 12 h.

In one embodiment, said administering at least twice daily provides a coefficient of variation, preferably a macrolide blood level coefficient of variation, below 40%, preferably below 35%, more preferably below 32%, even more preferably 30% or less, such as in a range of from 20% to 30% or in a range of from 25% to 30%. There is normally a physiologic, particularly biologic variation in patients that is dictated by factors such as the degree of hydration, diet, diurnal timing, and exercise. Furthermore, there is variation in drug responses between different patients. For example, the variation within one subject, particularly intra-subject variation, and/or the variation between subjects, particularly inter-subject variation, may be quantified with a coefficient of variation. The term "coefficient of variation", as used herein, preferably relates to an intra-subject and/or an inter-subject variation of pharmacokinetic parameters such as a macrolide blood level. In one embodiment, the variation is an intra-subject variation; for example an intra-subject macrolide blood level variation. In one embodiment, the coefficient of variation is a coefficient of macrolide blood level variation, e.g. inter-subject macrolide blood level variation and/or intra-subject macrolide blood level variation. In one embodiment, the coefficient of variation is an intra-subject and/or inter-subject coefficient of variation of pharmacokinetic measures, preferably an intra-subject coefficient of variation of pharmacokinetic measures. The coefficient of variation (CV) may be defined as the ratio of the standard deviation to the mean. Advantageously, the intra-subject and inter-subject variation is reduced with the macrolide for use of the invention compared to a standard oral administration of macrolide, such as a Prograf® administration. For example, inter-individual pharmacokinetic variation can be affected by the time passed since the transplant, patient demographics (age and race), hepatic and renal function, the hematocrit level, food administration, concomitant medications (corticosteroids, antifungals, calcium channel blockers, etc.), and the genotype for metabolic enzymes.

In one embodiment, said administering at least twice daily provides

- a $t_{max}$ in a range of from about 3 h to about 10 h after said first time point, such as in a range of from about 4 h to about 10 h after said first time point, preferably in a range of from about 7 h to about 9 h after said first time point, more preferably in a range of from about 7.5 h to about 8.5 h after said first time point; and/or
- a $t_{max}$ in a range of from about 1 h to about 6 h after said second time point, preferably in a range of from about 2 h to about 5 h after said second time point, more preferably in a range of from about 3.5 h to about 4.5 h after said second time point, e.g. about 4 h after said second time point.

$T_{max}$ is typically the time to a peak drug concentration, particularly the time it takes for a drug to reach a maximum concentration ($C_{max}$) after administration of a drug. Advantageously, the macrolide for use of the invention allows to achieve very high blood levels with relatively low doses, e.g. in a range of from about 1 h to about 6 h after said second time point.

In one embodiment, said administering at least twice daily provides

- a $C_{max}$ in a range of from about 0.05 ng/mL to about 50 ng/mL, preferably in a range of from about 0.1 ng/mL to about 30 ng/mL, more preferably in a range of from about 0.7 ng/mL to about 20 ng/mL, even more preferably in a range of from about 5.5 ng/mL to about 20 ng/mL; and/or
- a $C_{trough}$ in a range of from about 0.01 ng/mL to about 18 ng/mL, preferably in a range of from about 0.5 ng/mL to about 16 ng/mL, more preferably in a range of from about 3 ng/mL to about 15 ng/mL, such as in a range of from about 3 ng/mL to about 7 ng/mL or in a range of from about 5 ng/mL to about 7 ng/mL.

In a preferred embodiment, $C_{max}$ is 20 ng/mL or less and/or $C_{trough}$ is 5 ng/mL or more. For example, an efficient and safe prevention and treatment may be achieved if $C_{max}$ is 20 ng/mL or less and $C_{trough}$ is 5 ng/mL or more.

In one embodiment, said administering at least twice daily provides a whole blood concentration in a range of from 0.1 ng/mL to about 20 ng/mL, preferably in a range of from 0.2 ng/mL to about 10 ng/mL, more preferably in a range of from 0.3 ng/mL to about 7 ng/mL, at a time point in a range of from about 1 h to about 4 h, preferably from about 1.5 h to about 3.5 h, after said first time point. In one embodiment, said administering at least twice daily provides

- an $AUC_{0-4}$ in a range of from about 0.5 h*ng/mL to about 7.0 h*ng/mL, preferably in a range of from about 1.0 h*ng/mL to about 6.5 h*ng/mL;
- an $AUC_{4-8}$ in a range of from about 0.7 h*ng/mL to about 15.0 h*ng/mL, preferably in a range of from about 1.5 h*ng/mL to about 12.0 h*ng/mL;
- an $AUC_{0-24}$ in a range of from about 4.0 h*ng/mL to about 200.0 h*ng/mL, preferably in a range of from about 5.0 h*ng/mL to about 60.0 h*ng/mL, more preferably in a range of from about 10.0 h*ng/mL to about 55.0 h*ng/mL;
- an $AUC_{0-tlast}$ in a range of from about 4.0 h*ng/mL to about 250.0 h*ng/mL, preferably in a range of from about 5.0 h*ng/mL to about 70.0 h*ng/mL, more preferably in a range of from about 10.0 h*ng/mL to about 60.0 h*ng/mL;
- a half life in a range of from about 12 h to about 16 h, preferably of from about 13.5 h to about 15.5 h, more preferably of from about 14 h to about 15 h; and/or

- an elimination rate in a range of from about 0.011/h to about 0.08 1/h, preferably in a range of from 0.04 1/h to about 0.05 1/h, even more preferably in a range of from 0.045 1/h to about 0.049 1/h.

In one embodiment, said administering at least twice daily is performed for a duration of at least 2 days, preferably at least 1 week, more preferably at least 1 month, even more preferably at least 1 year. Thus, for example, said first and second doses may be administered daily for at least 2 days, preferably at least 1 week, more preferably at least 1 month, even more preferably at least 1 year. In one embodiment, if said macrolide is for use in preventing or treating a chronic immunological disease or disorder, such as a transplant rejection, said administering at least twice daily may be performed for a lifetime of a patient. Advantageously, by mucosally, particularly buccally, administering said macrolide for use, the side effects may be reduced, since lower doses are needed to achieve a blood level in the therapeutic window, and the life quality of the patient is enhanced.

In one embodiment, the term "patient" relates to a human or an animal, preferably a human. In one embodiment, said patient is not a fasting patient. Advantageously, the macrolide for use, particularly when buccally administered, achieves desired blood levels, such as blood levels in the therapeutic window, even if the patient is not a fasting patient. In one embodiment, the patient is not a fasting patient. In one embodiment, the patient is a pediatric patient, a juvenile patient, or an adult patient. The adult patient may be a geriatric patient. The terms "patient" and "subject" may be used interchangeably.

The term "mucoadhesive layer", as used herein, relates to a layer that adheres to a mucosa and/or that is configured to adhere to a mucosa, e.g. the oral and/or buccal mucosa. In a preferred embodiment, the mucoadhesive layer is provided in the form of a mucoadhesive film, particularly in the form of a mucoadhesive film comprising said mucoadhesive layer and optionally further layer(s). Preferably, the mucoadhesive layer comprises said macrolide. For example, the macrolide may be comprised by said layer in the form of nanoparticulate macrolide or non-nanoparticulate macrolide. In one embodiment, the mucoadhesive layer optionally comprises a surfactant, e.g. polysorbate. In one embodiment, the mucoadhesive layer is swellable in an aqueous liquid, but is insoluble in the aqueous liquid and/or has negligible solubility in the aqueous liquid. The insolubility or low solubility increases the adhesive time on the mucosa and thus enables that the active ingredient is released over a long period of time, e.g. for at least 60 min. The term "mucoadhesive buccal layer" or "MBF", as used herein, relates to a mucoadhesive layer which is configured for a buccal application. In one embodiment, said macrolide is present in said mucoadhesive layer at a concentration of at least 2 wt%, preferably at least 3 wt%, e.g. 3.2 wt%. In one embodiment, said macrolide is administered in the form of a mucoadhesive layer comprising a mucoadhesive polymer. In one embodiment, said mucoadhesive layer comprises a mucoadhesive polymer. In one embodiment, said mucoadhesive layer or mucoadhesive film comprises a therapeutically effective amount of said macrolide.

In one embodiment, the mucoadhesive layer comprises a mucoadhesive polymer and/or a cellulose derivative, and further comprises said macrolide, e.g. nanoparticulate macrolide or non-nanoparticulate macrolide. In one embodiment, the mucoadhesive layer comprises a mucoadhesive polymer such as alginate. In one embodiment, the mucoadhesive layer comprises alginate. In one embodiment, the mucoadhesive layer is an alginate layer. In one embodiment, the mucoadhesive layer comprises non-nanoparticulate macrolide, preferably micronized crystalline non-nanoparticulate macrolide and/or molecularly dissolved non-nanoparticulate macrolide. In one embodiment, said non-nanoparticulate macrolide is present in said layer in the form of micronized macrolide, preferably micronized crystalline macrolide, and/or in a molecularly dissolved non-nanoparticulate form. In one embodiment, said micronized macrolide has an average particle size of from 1 to 100 $\mu$m, preferably of from 1.5 to 50 $\mu$m, more preferably of from 2 to 25 $\mu$m. In one embodiment, said non-nanoparticulate macrolide is present in said mucoadhesive layer in the molecularly dissolved non-nanoparticulate form; and, optionally, said mucoadhesive polymer and/or said cellulose derivative, if present, is/are a polymer soluble in an organic solvent.

In one embodiment, the mucoadhesive layer comprises

a) a mucoadhesive polymer, preferably an amphiphilic polymer; hydroxypropyl cellulose (HPC); carboxymethyl cellulose (CMC); a plasticizer, preferably glycerol; and optionally a colorant, preferably $TiO_2$; preferably 10 wt% - 30 wt% of mucoadhesive polymer; 25 wt% - 35 wt% of hydroxypropyl cellulose (HPC); 10 wt% - 25 wt% of carboxymethyl cellulose (CMC); 10 wt% - 16 wt% of said plasticizer; and optionally 1 wt% - 4 wt% of said colorant; or
b) a mucoadhesive polymer, preferably selected from crosslinked polyacrylic acid polymers and copolymers of methyl vinyl ether and maleic anhydride; hydroxypropyl cellulose (HPC); ethyl cellulose (EC); a plasticizer, preferably glycerol; and optionally a colorant, preferably $TiO_2$; preferably 2 wt% - 17 wt% of mucoadhesive polymer; 30 wt% - 70 wt% of hydroxypropyl cellulose (HPC); 5 wt% - 40 wt% of said ethyl cellulose; 3 wt% - 20 wt% of said plasticizer; and optionally 1 wt% - 5 wt% of said colorant.

The term "nanoparticulate macrolide", as used herein, relates to the macrolide being in the form of nanoparticles, particularly nanoparticles having a size in the range of 1 nm to 1000 nanometers. In one embodiment, the mucoadhesive layer comprises said macrolide in the form of nanoparticles, e.g. nanoparticles having a particle size of about 10 to about 400 nm. In one embodiment, the mucoadhesive layer comprises macrolide nanoparticles. For example, nanoparticles may have a polydispersity index of ≤ about 0.4. Nanoparticles may comprise a stabilizing agent and/or a surfactant. For example, the stabilizing agent may comprise polyvinyl pyrrolidone, vinyl pyrrolidone-/vinylacetate-copolymer, polyethylenglycol and/or a cellulose derivative such as hydroxypropylmethyl cellulose (HPMC), hydroxypropylmethyl cellulose phthalate (HPMCP), hydroxypropylmethyl cellulose acetate succinate (HPMCAS), hydroxypropyl cellulose (HPC), and/or carboxymethyl cellulose (CMC). In one embodiment, the mucoadhesive layer comprises nanoparticulate macrolide and/or non-nanoparticulate macrolide.

The term "non-nanoparticulate macrolide", as used herein, relates to the macrolide not being in the form of nanoparticles. In one embodiment, non-nanoparticulate macrolide relates to macrolide that is not formulated as nanoparticles, particularly not formulated as nanoparticles having a size in the range of 1 nm to 1000 nanometers. In one embodiment, non-nanoparticulate macrolide is macrolide which is formulated as molecularly dissolved macrolide and/or micronized crystalline macrolide. In one embodiment, molecularly dissolved macrolide is not molecularly dissolved macrolide in the form of nanoparticles, e.g. is not molecularly dissolved macrolide present in a micelle or liposome. In one embodiment, molecularly dissolved macrolide does not relate to nanoparticles comprising molecularly dissolved macrolide. In one embodiment, molecularly dissolved macrolide is formulated in the form of a solid solution, e.g. embedded and/or dissolved in a polymer matrix. In one embodiment, micronized crystalline macrolide is not in the form of nanoparticles, e.g. is not in the form of nanoparticles comprising micronized crystalline macrolide. In one embodiment, micronized crystalline macrolide has an average particle size of > 1000 nm. In one embodiment, micronized crystalline macrolide is in the form of microparticles, preferably having an average particle size, in the range of 1 $\mu$m 1000 $\mu$m, preferably > 1000 nm. In one embodiment, non-nanoparticulate macrolide comprised by a mucoadhesive layer and/or mucoadhesive film is not in the form of nanoparticles and/or is not comprised by nanoparticles. In one embodiment, said non-nanoparticulate macrolide is present in said layer in the form of micronized macrolide, preferably micronized crystalline macrolide, in a molecularly dissolved non-nanoparticulate form, and/or in the form of crystalline macrolide; preferably said non-nanoparticulate macrolide is present in said layer in the form of micronized macrolide, preferably micronized crystalline macrolide, and/or in a molecularly dissolved non-nanoparticulate form. In one embodiment, a mucoadhesive layer comprising macrolide in the form of micronized macrolide, preferably micronized crystalline macrolide, and/or in a molecularly dissolved non-nanoparticulate form, further comprises macrolide in a crystalline form.

The term "average particle size", as used herein, relates to the average diameter of an analyzed batch of particles, e.g. (micro)particles of micronized macrolide. For example, the average particle size may be the average diameter of a batch of particles, wherein, if the particles are not spherical, the longest extension of each particle is considered as the diameter. In one embodiment, the average particle size is in the range of from 1 to 100 $\mu$m, preferably of from 1.5 to 50 $\mu$m, more preferably of from 2 to 25 $\mu$m. In one embodiment, an average particle size, e.g. of a micronized macrolide, is measured using any technique known to a person skilled in the art, for example laser diffraction analysis or dynamic light scattering, preferably laser diffraction analysis. The average particle size is defined as the average diameter of the analyzed particles determined by a suitable process, e.g. using dynamic light scattering. In one embodiment, the average particle size of a micronized macrolide is 2 $\mu$m to 25 $\mu$m. In one embodiment, the micronized macrolide has a particle size distribution $D_{10}$ 2 $\mu$m; $D_{50}$ 10 $\mu$m; and $D_{90}$ 25 $\mu$m. In one embodiment, 10% of all particles had a particle size <2 $\mu$m, 50% of all particles had a particle size <10 $\mu$m, and 90% of all particles had a particle size <10 $\mu$m. In one embodiment, all particles had a particle size > 1 $\mu$m. In one embodiment, the micronized macrolide is not in the form of nanoparticles. In one embodiment, the average particle size is $D_{50}$. In one embodiment, the average particle size of micronized macrolide is > 1 $\mu$m, it being understood that, preferably, none of the particles is a nanoparticle. In one embodiment, the particle size of micronized macrolide is > 1 $\mu$m. In one embodiment, the particle size of each micronized macrolide particle is > 1 $\mu$m. The term "micronized", as used herein, relates to the average particle diameters being in the micrometer range and/or being reduced to the micrometer range, preferably by micronization. In one embodiment, "micronized crystalline macrolide" relates to crystalline macrolide having an average particle diameter in the micrometer range.

In one embodiment, the macrolide is formulated in the form of a mucoadhesive film; preferably is formulated in the form of a mucoadhesive film, comprising a mucoadhesive layer comprising said macrolide and comprising a further layer such as a backing layer. In one embodiment, the mucoadhesive layer is provided in the form of a mucoadhesive film. In one embodiment, said macrolide is administered in the form of a mucoadhesive film, preferably mucoadhesive buccal film. In one embodiment, the term "mucoadhesive film", as used herein, relates to a film that adheres to a mucosa and/or that is configured to adhere to a mucosa, e.g. the oral and/or buccal mucosa. In one embodiment, the terms "film", "mucoadhesive film", "mucoadhesive buccal film", and "MBF" are used interchangeably. In one embodiment, when referring to "mucoadhesive buccal film" or "MBF", such abbreviation does not only relate to a mucoadhesive buccal film, but also to a mucoadhesive film for other applications than a buccal application. In one embodiment, said mucoadhesive film, preferably mucoadhesive buccal film, comprises a mucoadhesive layer comprising macrolide, preferably a mucoadhesive layer as defined above; and further comprises a backing layer, preferably a backing layer impermeable for the macrolide and/or impermeable for water. In one embodiment, the backing layer comprises a plasticizer, preferably selected from glycerol, polyethylene glycol, e.g. low molecular weight polyethylene glycol such as PEG 200 or PEG 400, propylene glycol, sorbitol, triacetin, and tributylcitrate, more preferably glycerol; and a water-insoluble polymer and/or a cellulose derivative, e.g. hydroxyethyl cellulose, preferably a water-insoluble cellulose derivative, more preferably ethyl cellulose. The mucoadhesive layer or film is advantageous in that the rich blood circulation of the oral and/or buccal mucosa ensures a rapid transfer of the active substance into the blood circulation. Advantageously, the active ingredient is largely absorbed through the mucous membrane and thus the "first-pass metabolism", which occurs in the conventional delivery form of an active ingredient in tablet form, is avoided. Further, the mucoadhesive layer or film has the advantage that the macrolide is protected from degradation due to pH and digestive enzymes of the gastrointestinal tract. The mucoadhesive layer or film further provide(s) a rapid onset of action, e.g. relative to the oral route. A mucoadhesive layer or film is an easy way of drug administration and therefore especially suitable for use in pediatrics and geriatrics. Furthermore, the mucoadhesive layer or film avoids hurdles related to drug administration via the nasogastric tube and is flexible in physical shape, state, size, and surface. The mucoadhesive layer or film is further advantageous in that it allows an accurate dosing. In a preferred embodiment, when referring to a "mucoadhesive layer", a mucoadhesive layer in the form of a mucoadhesive film is meant, particularly a mucoadhesive film comprising said mucoadhesive layer and optionally further layer(s). In one embodiment, the mucoadhesive film comprises a mucoadhesive polymer such as alginate. In one embodiment, the mucoadhesive film comprises alginate. In one embodiment, the mucoadhesive film is an alginate film.

In one embodiment, the mucoadhesive film further comprises an intermediate layer arranged between the mucoadhesive layer and the backing layer; wherein, preferably, said intermediate layer facilitates adhesion between the mucoadhesive layer and the backing layer; wherein, optionally, said intermediate layer comprises a plasticizer, preferably selected from glycerol, polyethylene glycol, e.g. low molecular weight polyethylene glycol such as PEG 200 or PEG 400, propylene glycol, sorbitol, triacetin, and tributylcitrate, more preferably glycerol; and a polymer, preferably a polymer comprising polyvinyl acetate and/or polyvinylpyrrolidone, more preferably a vinylpyrrolidone-vinyl acetate copolymer.

In one embodiment, the mucoadhesive film, preferably mucoadhesive buccal film, comprises a mucoadhesive layer, a backing layer, and an intermediate layer,

wherein said intermediate layer is arranged between the mucoadhesive layer and the backing layer, and wherein said mucoadhesive layer comprises a macrolide, preferably tacrolimus; wherein, preferably, said intermediate layer facilitates adhesion between the mucoadhesive layer and the backing layer;
wherein, optionally, said intermediate layer comprises a plasticizer, preferably selected from glycerol, polyethylene glycol, e.g. low molecular weight polyethylene glycol such as PEG 200 or PEG 400, propylene glycol, sorbitol, triacetin, and tributylcitrate, more preferably glycerol;
wherein, optionally, said intermediate layer comprises a polymer, preferably a polymer comprising polyvinyl acetate and/or polyvinylpyrrolidone, more preferably a vinylpyrrolidone-vinyl acetate copolymer.

In one embodiment, the mucoadhesive film is a solid preparation comprising a single or multiple layers of suitable material(s) intended to be applied to a body cavity, preferably the buccal cavity, to obtain a systemic effect or a local effect, preferably a systemic effect; and/or the mucoadhesive film is a flexible single-dose preparation configured to be applied to a body cavity, preferably the oral cavity, to obtain either a systemic or a local effect by delivering the macrolide over a certain period of time, after which it is then removed. In one embodiment, the mucoadhesive film is an oromucosal patch.

In one embodiment, the mucoadhesive film comprises said mucoadhesive layer and optionally a backing layer. In a preferred embodiment, the mucoadhesive film comprises said mucoadhesive layer and a backing layer. In one embodiment, the mucoadhesive film comprises said mucoadhesive layer; a backing layer, preferably an impermeable backing layer; and optionally an intermediate layer. In one embodiment, the mucoadhesive layer contains the macrolide, e.g. in a polymer matrix or in nanoparticles, and provides prolonged contact of the macrolide with the buccal mucosa. In one embodiment, the film comprises at least two layers, for example two layers laminated to each other; preferably a mucoadhesive layer containing the active ingredient and a backing layer. In one embodiment, to prevent a release of the macrolide to the oral cavity, the backside of the mucoadhesive layer is sealed with a backing layer, preferably a water-insoluble and/or macrolide impermeable backing layer. In one embodiment, the backside of the mucoadhesive layer is the side configured to be facing the oral cavity. In one embodiment, the two layers are attached to each other via an intermediate layer, preferably an adhesive layer. In one embodiment, the mucoadhesive layer is configured to be administered to a patient, e.g., by incorporating the mucoadhesive layer in a mucoadhesive film, an oromucosal patch, or any other application system.

In one embodiment, the adhesion characteristic of the mucoadhesive layer and/or film is sufficient for an application of at least 30 min, preferably at least 60 min, i.e. the layer and/or film adheres to the mucosa for at least 30 min, preferably at least 60 min. In one embodiment, ethyl cellulose is comprised by a mucoadhesive layer and/or film and allows for an application time of at least 30 min, preferably at least 60 min, due to its low water solubility. In one embodiment, the backing layer comprises ethyl cellulose. In one embodiment, the mucoadhesive film is a non-dissolvable film that must be removed from the mucosa, e.g. buccal mucosa, after drug release. In one embodiment, the mucoadhesive layer is applied to a patient for a period of at least 30 min.

In one embodiment, the mucoadhesive layer and/or the mucoadhesive film has/have an area of 0.5 cm$^2$ to 10 cm$^2$, preferably 2 cm$^2$ to 8 cm$^2$. In one embodiment, the area weight of the mucoadhesive film is about 20 g/m$^2$ to about 300 g/m$^2$, preferably about 50 g/m$^2$ to about 260 g/m$^2$. In one embodiment, the mucoadhesive film has a film thickness of about 20 $\mu$m to about 1000 $\mu$m, preferably of about 50 $\mu$m to about 500 $\mu$m, more preferably of about 50 $\mu$m to about 300 $\mu$m, e.g. about 150 $\mu$m to about 260 $\mu$m.

In one embodiment, said mucoadhesive layer has an area weight in the range of from 70 to 180 g/m$^2$, preferably in the range of from 100 to 150 g/m$^2$; and/or

said backing layer has an area weight in the range of from 40 to 100 g/m$^2$, preferably in the range of from 40 to 60 g/m$^2$, e.g. 50 g/m$^2$; and/or
said intermediate layer, if present, has an area weight in the range of from 40 to 100 g/m$^2$, preferably in the range of from 40 to 70 g/m$^2$, e.g. 60 g/m$^2$.

In one embodiment, at least one of the layers comprises a colorant, e.g. a white pigment is comprised by the mucoadhesive layer and a blue dye is comprised by the backing layer. Advantageously, a colorant in at least one of the layers allows to distinguish between the adhesive layer and the backing layer. In one embodiment, the mucoadhesive film further comprises a flavoring agent and/or sweetening agent, such as an agent selected from citric acid, peppermint oil, sodium saccharin, and citrus flavor. Advantageously, the flavoring agent and/or sweetening agent enhances the mouthfeel for the patient. Particularly, such flavoring agent and/or sweetening agent efficiently mask(s) an unpleasant taste of the macrolide or other MBF components. In one embodiment, at least one layer further comprises at least one adjuvant selected from the group comprising stabilizing agents, e.g. chelators, colorants, flavorings, sweeteners, taste masking agents, emulsifiers, enhancers, pH regulators, humectants, preservatives and/or antioxidants. In one embodiment, at least one layer comprises a chelator, preferably EDTA. In one embodiment, when referring to a "layer", the mucoadhesive layer, the intermediate layer, and/or the backing layer are meant. In a preferred embodiment, a mucoadhesive layer is provided in the form of a mucoadhesive film.

In one embodiment, the dose strength of the film is determined by the size and/or area of the film, wherein, optionally, the quantitative composition per film area is constant. In one embodiment, the film comprises a dose of up to 250 mg macrolide, e.g. of up to 50 mg macrolide. In one embodiment, an amount of macrolide is measured using HPLC. In one embodiment, HPLC is used to quantify a macrolide. In one embodiment, the mucoadhesive film is administered and/or configured to be administered to adult patients or pediatric patients, e.g. children of the age up to about 18 years.

In one embodiment, said mucoadhesive layer and/or backing layer comprise(s) a colorant. In one embodiment, said mucoadhesive layer and said backing layer each comprise a colorant, wherein said mucoadhesive layer comprises a first colorant, e.g. TiO$_2$, and said backing layer comprises a second colorant, e.g. brilliant blue, wherein said first colorant and said second colorant are different from each other. In one embodiment, the mucoadhesive layer and/or the mucoadhesive film has/have a water content of < 5 wt%, preferably < 3 wt%, more preferably < 2 wt%.

The term "plasticizer", as used herein, relates to any plasticizer known to a person skilled in the art, preferably plasticizers which are suitable for in vivo application. In one embodiment, the plasticizer, e.g. a plasticizer comprised by a mucoadhesive layer, an intermediate layer, and/or a backing layer, is selected from glycerol, poly(ethylene glycol), preferably low molecular weight poly(ethylene glycol) such as PEG 200 or PEG 400, propylene glycol, sorbitol, triacetin, and tributylcitrate. In one embodiment, low molecular weight poly(ethylene glycol) has a molecular weight $\leq$ 600 daltons, preferably $\leq$ 450 daltons, e.g. 420 daltons. A plasticizer allows for advantageous mechanical properties of the layer(s).

The term "backing layer", as used herein, relates to a layer of a mucoadhesive film. In one embodiment, the backing layer covers the backside of a mucoadhesive layer in a mucoadhesive film. The backside is preferably the side of the mucoadhesive layer or film configured to be facing the body cavity, preferably oral cavity, i.e. the side facing away from the mucosa. In one embodiment, the backing layer is impermeable for macrolide and/or impermeable for water. Advantageously, the backing layer prevents macrolide release from the mucoadhesive layer into a body cavity, e.g. into the oral cavity. In one embodiment, the backing layer covers the backside of the mucoadhesive layer and optionally further covers the side regions of the mucoadhesive layer. In one embodiment, the backing layer is a sealing layer. In one embodiment, the backing layer, preferably impermeable backing layer, prevents undesired release of the macrolide into the body cavity, preferably oral cavity. In one embodiment, the backing layer is configured to ensure unidirectional release of the macrolide into the mucosa, preferably buccal mucosa. In one embodiment, the backing layer prevents release of the macrolide into the buccal cavity. Advantageously, by preventing the release into the cavity, side effects are reduced. In one embodiment, the backing layer is insoluble in water and/or an aqueous liquid such as saliva. In one embodiment, the backing layer does not dissolve in water and/or an aqueous liquid such as saliva for a period of 30 min to 75 min, preferably at least 60 min. In one embodiment, the backing layer and/or backing layer polymer mixture comprise(s) a water-insoluble polymer and/or a cellulose derivative, e.g. hydroxyethyl cellulose, preferably a water-insoluble cellulose derivative, more preferably ethyl cellulose, and thus the dissolution of the backing layer in the oral cavity is prevented. In one embodiment, the backing layer and/or backing layer polymer mixture comprise(s) a water-insoluble polymer and/or a polymer having low water solubility, preferably a water-insoluble polymer such as ethyl cellulose. In one embodiment, the backing layer polymer mixture comprises at least one component, preferably all components of a backing layer. In one embodiment, the backing layer is organic solvent based, e.g. ethanol based, and/or the mucoadhesive layer is water based. In one embodiment, the backing layer comprises a polymer comprising polyvinyl acetate and/or polyvinylpyrrolidone, preferably comprising polyvinyl acetate. In one embodiment, the backing layer comprises a colorant, e.g. brilliant blue. In one embodiment, the backing layer comprises a plasticizer, e.g. triacetin.

The term "water-insoluble polymer", as used herein, relates to a polymer which is not soluble in water, e.g. ethyl cellulose and poly(methyl methacrylate). The term "polymer having low water solubility", as used herein, relates to a polymer which has negligible and/or low solubility in water, e.g. hydroxyethyl cellulose. In one embodiment, a "water-insoluble polymer" and a "polymer having low water solubility" are polymers that prevent a dissolution of the mucoadhesive film during its application in a body cavity, preferably buccal cavity, for at least 30 min. In one embodiment, the water-insoluble polymer and/or the polymer having low water solubility are selected such that a film application time of at least 15 min, preferably of at least 30 min is provided, preferably without dissolution of the film. In one embodiment, the water-insoluble polymer is ethyl cellulose and/or poly(methyl methacrylate). In one embodiment, the cellulose derivative comprised by a backing layer is hydroxyethyl cellulose and/or ethyl cellulose. In one embodiment, the backing layer is configured to be insoluble in an aqueous liquid for at least 30 min, preferably at least 60 min. In one embodiment, the backing layer is configured to be impermeable for a macrolide for at least 30 min, preferably at least 60 min; preferably configured to be impermeable for a macrolide for at least 30 min, preferably at least 60 min in an aqueous liquid, such as saliva. In one embodiment, the backing layer and/or backing layer polymer mixture comprise(s) polymers that are insoluble in an aqueous liquid for at least 30 min. In one embodiment, the backing layer comprises ethyl cellulose, hydroxyethyl cellulose, and/or poly(methyl methacrylate), and optionally further comprises a plasticizer. In one embodiment, the backing layer comprises ethyl cellulose and/or poly(methyl methacrylate), and optionally further comprises a plasticizer.

The term "intermediate layer", as used herein, relates to a layer of a mucoadhesive film, preferably a layer arranged between a mucoadhesive layer and a backing layer. In one embodiment, the intermediate layer is an adhesive layer. In one embodiment, the intermediate layer facilitates adhesion of the mucoadhesive layer and the backing layer. In one embodiment, the intermediate layer prevents migration of the macrolide into the backing layer. In one embodiment, a film comprises an intermediate layer between the mucoadhesive layer and the backing layer to prevent migration of macrolide into the backing layer. In one embodiment, the terms "adhesive interlayer" and "intermediate layer" are used interchangeably. In one embodiment, a mucoadhesive layer and a backing layer are connected via an intermediate layer. For example, a film comprising said backing layer and said intermediate layer may be combined with the mucoadhesive layer after drying the layer(s) to prevent migration of macrolide into the adhesive interlayer and/or backing layer. Advantageously, incorporating an intermediate layer in a mucoadhesive film allows to prevent dissolution of the macrolide in the backing layer during the method of preparing the film. Advantageously, the intermediate layer prevents that the amount of macrolide in the mucoadhesive layer decreases and/or that the macrolide degrades during a process of preparing a backing layer on a mucoadhesive layer. Advantageously, the intermediate layer prevents that macrolide is dissolved in ethanol comprised by a backing layer. In one embodiment, a mucoadhesive film comprises an intermediate layer, e.g. a film comprising non-nanoparticulate macrolide in the form of micronized macrolide, preferably micronized crystalline macrolide, and/or in a molecularly dissolved non-nanoparticulate form. In one embodiment, the intermediate layer comprises a polymer, optionally an adhesive polymer. In one embodiment, the intermediate layer comprises a polymer comprising polyvinyl acetate and/or polyvinylpyrrolidone; for example selected from poly(vinylpyrrolidone), poly[(vinylpyrrolidone)-co-(vinyl acetate)], e.g. a copolymer of 6 parts polyvinyl pyrrolidone and 4 parts polyvinyl acetate, and a mixture of poly(vinylacetate) and poly(vinyl pyrrolidone), e.g. a 8:2 mixture of poly(vinylacetate) and poly(vinyl pyrrolidone). In one embodiment, the intermediate layer comprises a plasticizer, e.g. glycerol.

The term "solid solution", as used herein, relates to the macrolide being dissolved on a molecular level in a diluent or matrix in the solid state, preferably being dissolved on a molecular level in a polymer matrix. In one embodiment, the term "solid solution" relates to the macrolide being dissolved, and optionally embedded, in a polymer matrix. In one embodiment, macrolide in a molecularly dissolved non-nanoparticulate form relates to a solid solution of the macrolide. In one embodiment, the terms "solid solution" and "molecularly dissolved non-nanoparticulate form" are used interchangeably. In one embodiment, a solid solution is molecularly dissolved non-nanoparticulate macrolide provided in a polymer matrix, preferably comprising at least one polymer soluble in an organic solvent. In one embodiment, molecularly dissolved non-nanoparticulate macrolide is provided in a polymer matrix, preferably comprising at least one polymer soluble in an organic solvent. In one embodiment, a polymer soluble in organic solvents, e.g. a copolymer of methyl vinyl ether and maleic anhydride, is used to achieve an appropriate mucoadhesion of a water-free mucoadhesive film formulation, e.g. for a solid solution. In one embodiment, molecularly dissolved non-nanoparticulate macrolide is in the form of a solid solution of macrolide. In one embodiment, the polymer soluble in an organic solvent is any polymer soluble in an organic solvent selected from a) amphiphilic polymers and hydrophilic polymers, b) poly(methacrylates), c) crosslinked polyacrylic acid polymers, d) copolymers of methyl vinyl ether and maleic anhydride, e) polymers comprising polyvinyl acetate and/or polyvinylpyrrolidone, f) cellulose derivatives, and combinations thereof. In one embodiment, the polymer soluble in an organic solvent is an amphiphilic or hydrophilic polymer, e.g. Soluplus®; a poly(methacrylate), e.g. a methacrylic acid copolymer; a cellulose derivative, preferably hydroxypropyl cellulose (HPC) or ethyl cellulose (EC), more preferably HPC; a crosslinked polyacrylic acid polymer, e.g. Carbopol®; a copolymer of methyl vinyl ether and maleic anhydride, e.g. Gantrez™; and/or a polymer comprising polyvinyl acetate and/or polyvinylpyrrolidone, e.g. Kollidon®. In one embodiment, the solid solution of macrolide comprises HPC; a crosslinked polyacrylic acid polymer, e.g. Carbopol®; a copolymer of methyl vinyl ether and maleic anhydride, e.g. Gantrez™; and/or a polymer comprising polyvinyl acetate and/or polyvinylpyrrolidone, e.g. Kollidon®. In one embodiment, the solid solution comprises HPC; and a crosslinked polyacrylic acid polymer, e.g. Carbopol®, and/or a copolymer of methyl vinyl ether and maleic anhydride, e.g. Gantrez™. In one embodiment, said non-nanoparticulate macrolide is present in said mucoadhesive layer in the form of micronized macrolide, preferably micronized crystalline macrolide, and/or in a molecularly dissolved non-nanoparticulate form, preferably a solid solution of molecularly dissolved macrolide. A highly advantageous macrolide blood profile can be achieved with such a mucoadhesive layer. The term "molecularly dissolved non-nanoparticulate form", as used herein, relates to the macrolide being dissolved on a molecular level, e.g. dissolved on a molecular level in a polymer matrix. In one embodiment, the mucoadhesive layer comprises at least one component, preferably polymer, selected from a) amphiphilic polymers and hydrophilic polymers, b) poly(methacrylates), c) crosslinked polyacrylic acid polymers, d) copolymers of methyl vinyl ether and maleic anhydride, e) polymers comprising polyvinyl acetate and/or polyvinylpyrrolidone, f) cellulose derivatives, and combinations thereof; preferably comprises at least one component selected from a)-e), and further comprises a cellulose derivative; wherein, preferably, if said layer comprises molecularly dissolved non-nanoparticulate macrolide, said at least one component is a polymer soluble in an organic solvent, preferably ethanol, acetone, and/or isopropanol. In one embodiment, the mucoadhesive layer comprises a plasticizer. In one embodiment, the mucoadhesive layer comprises non-nanoparticulate micronized crystalline macrolide, and/or molecularly dissolved non-nanoparticulate macrolide.

In one embodiment, if said non-nanoparticulate macrolide is present in said layer in the molecularly dissolved non-nanoparticulate form, the mucoadhesive polymer and/or cellulose derivative is a film-forming polymer, preferably a film-forming polymer soluble in an organic solvent, such as ethanol. In one embodiment, if said non-nanoparticulate macrolide is present in said layer in the molecularly dissolved non-nanoparticulate form, the molecularly dissolved non-nanoparticulate form is solubilized in an organic solvent. In one embodiment, if said non-nanoparticulate macrolide is present in said layer in the molecularly dissolved non-nanoparticulate form, the cellulose derivative is selected from HPC, EC, and combinations thereof. In one embodiment, if said non-nanoparticulate macrolide is present in said layer in the molecularly dissolved non-nanoparticulate form, the mucoadhesive polymer is selected from amphiphilic or hydrophilic polymers, e.g. Soluplus®; crosslinked polyacrylic acid polymers, e.g. Carbopol®; copolymers of methyl vinyl ether and maleic anhydride, e.g. Gantrez™; polymers comprising polyvinyl acetate and/or polyvinylpyrrolidone, preferably polymers comprising polyvinyl acetate and/or polyvinylpyrrolidone, e.g. Kollidon®; and cellulose derivatives, e.g. HPC, EC, and combinations thereof.

In one embodiment, for molecularly dissolving the macrolide, the macrolide is dissolved in the organic solvent in a concentration of about 10 mg/ml to about 500 mg/ml. The term "organic solvent", as used herein, relates to any organic solvent known to a person skilled in the art. In one embodiment, the organic solvent is an ICH class 3 or ICH class 2 solvent. In one embodiment, the organic solvent is non-toxic and/or volatile. In one embodiment, an organic solvent is selected from ethanol, methanol, acetone, tetrahydrofuran, acetic acid, acetonitrile, anisole, 1-Butanol, 2-Butanol, butyl acetate, chloroform, cyclohexan, 1,1,-diethoxypropane, 1,1-dimethoxymethane, 1,2-dimethoxyethane, 1,4-dioxane, 2,2-dimethoxypropane, dichlormethan, diethyl ether, di-iso-propyl ether, dimethyl sulfoxide, dimethylformamide, 2-ethoxyethanol, ethyl acetate, ethyl formate, ethylenglycol (1,2-Ethandiol), formic acid, heptane, hexane, isobutyl acetate, isopropyl acetate, 2-methoxyethanol, 2-methyl-1-propanol, 3-methyl-1-butanol, 1-methyl-2-pyrrolidone, methyl acetate, methyl t-butyl ether, methylbuylketon, methylcyclohexane, methylethyl ketone (MEK), methylisobutyl ketone, methylisopropyl ketone, methylthetrahydrofuran, n-methylpyrrolidone, 1-pentanol, 1-propanol, 2-propanol, pentane, petroleum ether, propyl acetate, pyridine, sulfolane, t-butyl alcohol, 2,2,4-trimethylpentan (i-Octan), toluol, trichloroacetic acid, trichloroethylene, trifluroacetic acid, xylol, and combinations thereof; preferably is selected from acetic acid, acetone, anisole, 1-butanol, 2-butanol, butyl acetate, tert-butylmethyl ether, DMSO, ethanol, ethyl acetate, ethyl ether, ethyl formate, formic acid, heptane, isobutyl acetate, isopropyl acetate, methyl acetate, 3-methyl-1-butanol, methylethyl ketone, 2-methyl-1-porpanol, 2-methyltetrahydrofuran, pentane, 1-pentanol, 1-propanol, 2-propanol, propyl acetate, trimethylamine; more preferably is selected from ethanol, acetone, and isopropanol. In one embodiment, the term "micronized macrolide solution", as used herein, relates to a solution of a micronized macrolide, particularly a solution of a micronized macrolide which has been dissolved in an organic solvent. In one embodiment, the terms "micronized macrolide solution" and "macrolide solution" are used interchangeably.

The term "mucoadhesive polymer", as used herein, relates to a polymer that facilitates and/or mediates mucoadhesion. In one embodiment, a mucoadhesive polymer is selected from a) amphiphilic polymers and hydrophilic polymers, b) poly(methacrylates), c) crosslinked polyacrylic acid polymers, d) copolymers of methyl vinyl ether and maleic anhydride, e) polymers comprising poly(vinyl acetate) and/or poly(vinylpyrrolidone), and mucoadhesive cellulose derivatives. In one embodiment, the mucoadhesive polymer is selected from poly(acrylic acid), e.g. Carbopol® 934 or Carbopol® 971 NF; crosslinked poly(acrylic acid), e.g. polycarbophil; amylopectin, e.g. Proloc™ 15; poly(methacrylic acid), e.g. Eudragit® L100 or Eudragit® S100; poly(methacrylate), e.g. Eudragit® E100, Eudragit® RL, or Eudragit® RS; poly[(maleic anhydride)-co-(vinyl methyl ether)] or a salt thereof, e.g. Gantrez™ AN 119, Gantrez™ AN 139, Gantrez™ AN 149, Gantrez™ AN 169, or Gantrez™ MS-955; gelatine; polysaccharides, e.g. alginates, chitosan, xanthan gum, hyaluronic acid, pectin, or pullulan; cellulose derivatives, e.g. sodium carboxymethylcellulose (CMC), hydroxypropylmethylcellulose (HPMC), hydroxypropylcellulose (HPC), hydroxyethyl methyl cellulose (HEMC), hydroxyethylcellulose (HEC), or methylcellulose (MC); poly(vinyl pyrrolidone), e.g. Kollidon® 30LP or Kollidon® 90F; poly[(vinylpyrrolidone)-co-(vinyl acetate)], e.g. Kollidon® VA64; a mixture of poly(vinylacetate) and poly(vinyl pyrrolidone), e.g. Kollidon® SR; poly(vinyl alcohol); and poly(vinyl acetate).

The term "amphiphilic polymers", as used herein, relates to any amphiphilic polymer, preferably mucoadhesive amphiphilic polymer, known to a person skilled in the art. Typically, and amphiphilic polymer has a hydrophilic (polar) moiety and a hydrophobic (non-polar) moiety. In one embodiment, amphiphilic polymers are selected from polyvinyl caprolactam-polyvinyl acetate-polyethylene glycol graft copolymers, e.g. Soluplus®, polyoxyl castor oils, and D-$\alpha$-tocopherol-polyethylenglycol-succinate (TPGS).

In one embodiment, a hydrophilic polymer is a polymer that contains polar or charged groups. In one embodiment, these groups are non-ionic, anionic, cationic and/or zwitterionic. In one embodiment, the hydrophilic polymer is soluble in water. For example, the hydrophilic polymer can be selected from starch and starch derivatives, dextran, cellulose and cellulose derivatives, such as carboxymethylcellulose, hydroxypropylcellulose, hydroxyethyl cellulose, hydroxypropyl methyl cellulose, hydroxypropylethyl cellulose, sodium carboxymethyl cellulose, ethyl or propyl cellulose, polyacrylic acid, polyacrylate, polyvinylpyrrolidone, polyethylene glycol/polyvinyl alcohol copolymer, polyvinyl alcohol, polyethylene oxide polymers, polyethylene oxide/polyethylene glycol copolymers, polyacrylamide, polyethylene glycol, gelatin, collagen, alginate, pectin, pullulan, traganth, chitosan, alginic acid, arabinogalactan, galactomannan, agar-agar, agarose, carrageenan, shellac, natural gums and/or copolymers thereof.

In one embodiment, poly(methacrylates) are selected from neutral protonizable poly(methacrylates) and cationic poly(methacrylates), preferably are selected from copolymers of dialkylaminoethyl methacrylates and methacrylic acid ester(s), and copolymers of trialkylammonioethyl methacrylates and methacrylic acid ester(s), in particular copolymers of dimethylaminoethyl methacrylates and methacrylic acid esters, and copolymers of trimethylammonioethyl methacrylates and methacrylic acid esters. For example, a poly(methacrylate) may be Eudragit® L100, Eudragit® S100, Eudragit® E100, Eudragit® RL, or Eudragit® RS.

The term "crosslinked polyacrylic acid polymers", as used herein, relates to polyacrylic acid polymers and crosslinked polyacrylic acid polymers, preferably crosslinked polyacrylic acid polymers, known to a person skilled in the art, such as a homopolymers, copolymers, and interpolymers, e.g. acrylic acid crosslinked with allyl sucrose or allyl pentaerythritol, acrylic acid and $C_{10}$-$C_{30}$ alkyl acrylate crosslinked with allyl pentaerythritol, and carbomer homopolymer or copolymer that contains a block copolymer of polyethylene glycol and a long chain alkyl acid ester. For example, a crosslinked polyacrylic acid polymer may be Carbopol® 934 or Carbopol® 971 NF.

The term "copolymers of methyl vinyl ether and maleic anhydride", as used herein, relates to any copolymer of methyl vinyl ether and maleic anhydride/maleic acid known to a person skilled in the art, e.g. copolymers of monoalkyl esters of poly (methyl vinyl ether/maleic acid) with varying ester groups. In one embodiment, copolymers of methyl vinyl ether and maleic anhydride are in the form of an anhydride or in a hydrolysed form, such as a mixed sodium and calcium salt of methyl vinyl ether and maleic anhydride e.g. Gantrez™ MS-955 polymer. In one embodiment, the copolymers of methyl vinyl ether and maleic anhydride are selected from poly(methyl vinyl ether-co-maleic anhydride), poly(methyl vinyl ether-co-maleic acid), monoethyl ester of poly(methylvinyl ether/maleic acid), mixture of monoethyl ester of poly(methylvinyl ether/maleic acid) and monobutyl ester of poly(methylvinyl ether/maleic acid), and mixed sodium/calcium salts of poly(methylvinyl ether/maleic anhydride). In one embodiment, a copolymer of methyl vinyl ether and maleic anhydride, e.g. Gantrez™, is a mixed sodium and calcium salt of a copolymer of methyl vinyl ether and (hydrolyzed) maleic anhydride. In one embodiment, the copolymer of methyl vinyl ether and maleic anhydride is a complexing agent. For example, a copolymer of methyl vinyl ether and maleic anhydride may be Gantrez™ AN 119, Gantrez™ AN 139, Gantrez™ AN 149, Gantrez™ AN 169, Gantrez™ AN-903, Gantrez™ S-96, Gantrez™ S-97, Gantrez™ ES-225, Gantrez™ ES-425, or Gantrez™ MS-955.

The terms "polymers comprising polyvinyl acetate and/or polyvinylpyrrolidone" and "polymers comprising polyvinyl acetate and polyvinylpyrrolidone", as used herein, relate to any polymers comprising polyvinyl acetate and/or polyvinylpyrrolidone known to a person skilled in the art, e.g. poly(vinylpyrrolidone), poly(vinylacetate), poly[(vinylpyrrolidone)-co-(vinyl acetate)], and a mixture of poly(vinylacetate) and poly(vinyl pyrrolidone). In one embodiment, the polymer comprising polyvinyl acetate and/or polyvinylpyrrolidone is selected from poly(vinylpyrrolidone), poly(vinylacetate), poly[(vinylpyrrolidone)-co-(vinyl acetate)], e.g. a copolymer of 6 parts polyvinyl pyrrolidone and 4 parts polyvinyl acetate, and a mixture of poly(vinylacetate) and poly(vinyl pyrrolidone), e.g. a 8:2 mixture of poly(vinylacetate) and poly(vinyl pyrrolidone). In one embodiment, a polymer comprising polyvinyl acetate and/or polyvinylpyrrolidone comprises or consists of poly(vinylpyrrolidone). In one embodiment, a polymer comprising polyvinyl acetate and/or polyvinylpyrrolidone comprises or consists of a vinylpyrrolidone-vinyl acetate copolymer, particularly poly[(vinylpyrrolidone)-co-(vinyl acetate)]. In one embodiment, a polymer comprising polyvinyl acetate and/or polyvinylpyrrolidone comprises or consists of a mixture of poly(vinylacetate) and poly(vinylpyrrolidone). In one embodiment, a polymer comprising polyvinyl acetate and/or polyvinylpyrrolidone comprises one or more, preferably multiple, vinyl acetate monomers and/or one or more, preferably multiple, vinylpyrrolidone monomers. For example, polymers comprising polyvinyl acetate and/or polyvinylpyrrolidone may be Kollidon® 30LP, Kollidon® 90F, Kollidon® VA64, or Kollidon® SR.

The term "cellulose derivative", as used herein, relates to cellulose and its derivatives. In one embodiment, the cellulose derivative is preferably a cellulose derivative that swells rather than dissolves upon contact with water and/or saliva. In one embodiment, the cellulose derivative is selected from hydroxypropyl cellulose (HPC), carboxymethyl cellulose (CMC), e.g. sodium carboxymethyl cellulose (CMC), hydroxypropyl methylcellulose (HPMC), hydroxyethyl cellulose (HEC), methyl cellulose (MC), hydroxyethyl methyl cellulose (HEMC), ethyl cellulose (EC), and a combination thereof. In one embodiment, the mucoadhesive layer comprises a cellulose derivative which is a mucoadhesive cellulose derivative, e.g. selected from hydroxypropyl cellulose (HPC), carboxymethyl cellulose (CMC), hydroxypropyl methylcellulose (HPMC), hydroxyethyl cellulose (HEC), methyl cellulose (MC), and a combination thereof. In one embodiment, a cellulose derivative soluble in an organic solvent is HPC and/or EC. In one embodiment, CMC is soluble in water but not in an organic solvent.

When preparing a mucoadhesive film, the layers of the film can either be prepared individually and then joined, e.g. via an intermediate layer and/or via an adhesive agent, and/or can be prepared on top of each other, e.g., by first preparing the mucoadhesive layer and then subsequently coating the mucoadhesive layer with the backing layer, optionally by coating the mucoadhesive layer with the intermediate layer and the backing layer. For example, preparing the mucoadhesive film may comprise preparing the mucoadhesive layer, the backing layer, and optionally the intermediate layer individually, and subsequently joining the layers using lamination, e.g. thermo-lamination, or compression. For example, after casting the mucoadhesive layer and subsequent drying of the layer, the second layer, e.g. intermediate layer or backing layer, is casted on top of the mucoadhesive layer. In one embodiment, the first and second layers, e.g. mucoadhesive layer and intermediate layer or backing layer, are mounted onto each other. In one embodiment, the mucoadhesive layer is not completely dried before applying the intermediate layer and/or backing layer. In one embodiment, a roll can be used to compress the layers onto each other. In one embodiment, preparing a mucoadhesive film comprises preparing a mucoadhesive layer and the backing layer, and subsequently joining the mucoadhesive layer and backing layer, e.g., by compressing or adhesion. In one embodiment, preparing a mucoadhesive film comprises preparing a mucoadhesive layer, an intermediate layer, and a backing layer, and subsequently joining the layers, e.g., by compressing or adhesion.

In one embodiment, a step of preparing a mucoadhesive layer and a step of preparing a backing layer are followed by a step of joining said mucoadhesive layer and said backing layer by compression and/or lamination, e.g. thermo-lamination. Optionally, said mucoadhesive layer and/or said backing layer are dried or partially dried prior to said step of joining said mucoadhesive layer and said backing layer. In one embodiment, a step of preparing a mucoadhesive layer, a step of preparing an intermediate layer, and a step of preparing a backing layer are followed by a step of joining said mucoadhesive layer, said intermediate layer, and said backing layer by compression and/or lamination, e.g. thermo-lamination. Optionally, said mucoadhesive layer, said intermediate layer, and/or said backing layer are dried or partially dried prior to said step of joining said mucoadhesive layer, said intermediate layer, and said backing layer. In one embodiment, said joining is performed by compression and/or lamination, e.g. thermo-lamination.

In one embodiment, said backing layer is prepared on a surface of said mucoadhesive layer, e.g. by coating said polymer mixture on a surface of said mucoadhesive layer, or is prepared separately and is subsequently joined with said mucoadhesive layer, optionally joined via an intermediate layer. In one embodiment, if said mucoadhesive layer and said backing layer, optionally said intermediate layer, are prepared separately, said layers are prepared on a process liner. In one embodiment, if said mucoadhesive layer comprises micronized macrolide, preferably micronized crystalline macrolide, said backing layer is prepared on a surface of said mucoadhesive layer, e.g. by coating said polymer mixture on a surface of said mucoadhesive layer. In one embodiment, if said mucoadhesive layer comprises molecularly dissolved macrolide, said backing layer is prepared separately from said mucoadhesive layer, and the mucoadhesive layer and backing layer are subsequently joined after their preparation, optionally joined via an intermediate layer. In one embodiment, the mucoadhesive layer, the backing layer, and optionally the intermediate layer, are each prepared on a process liner, and are subsequently joined after their preparation.

In one embodiment, preparing a mucoadhesive film comprises a step of joining the mucoadhesive layer, the backing layer, and optionally the intermediate layer, e.g. by compression and/or lamination, e.g. thermo-lamination. In one embodiment, said mucoadhesive layer, backing layer, and optionally intermediate layer, are dried or partially dried prior to a step of joining the layers. In one embodiment, the mucoadhesive layer, backing layer, and optionally intermediate layer, are prepared separately and are subsequently joined after their preparation, and/or are prepared on top of each other.

The inventors have found that the negative effects of the gastrointestinal passage after oral application can be eliminated by the innovative buccal therapy system presented herein. Another positive finding from the human kinetics study is the low variability of the pharmacokinetic (PK) profiles of both comparison groups: The PK profiles of the high-dose group and the low-dose group vary on average by about 25%, which is a very low inter-patient variation for tacrolimus. The inventors have found that, advantageously, buccal application can positively influence a central deficit of oral therapy.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by $\pm20\%$, $\pm15\%$, $\pm10\%$, and for example $\pm5\%$. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

BRIEF DESCRIPTION OF THE FIGURES

The present invention is now further described by reference to the following figures.

All methods mentioned in the figure descriptions below were carried out as described in detail in the examples.

**Figure 1** shows the arithmetic mean whole blood concentration-time profile of tacrolimus (ng/mL; PK Set, N = 10). Treatment Group 1 (T1): two doses of tacrolimus MBF 8.8 mg; Treatment Group 2 (T2): two doses of tacrolimus MBF 12.0 mg.

**Figure 2** shows individual tacrolimus blood levels in 10 healthy volunteers (5 subjects per group), bid application, second dose after 4 hours.

**Figure 3** shows tacrolimus mean blood level profiles in 10 healthy volunteers (5 subjects per group), bid application, second dose after 4 hours.

**Figure 4** shows the individual PK-profiles within the different groups (5 mini pigs per group) with an asymmetric dosing regimen for buccal application in which the second dose is administered 3,4, or 5 hours after the first dose.

**Figure 5** shows the plot of the mean curves. The highest blood levels are obtained when the second dose unit is administered 3h after the administration of the first dose.

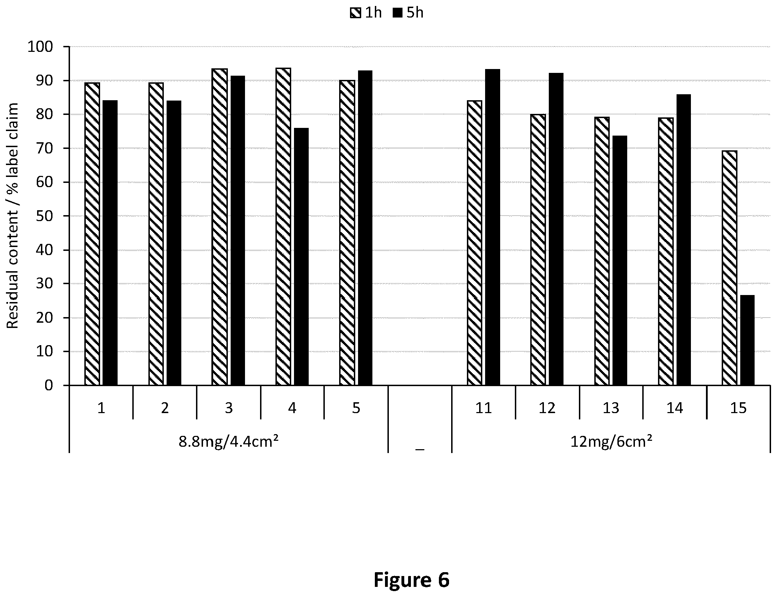

**Figure 6** shows the residual drug levels in the used films of a low dose group (loading with 8.8mg tacrolimus = 17.6 mg (bid)) and a high dose group (loading with 12 mg tacrolimus = 24 mg (bid)). Each subject was treated with a first dose in the form of a first mucoadhesive film at a first time point and with a second dose in the form of a second mucoadhesive film at a second time point about 4 hours after said first time point. Each of the films was removed after a duration of about 1 hour. The patterned bar shows the residual amount of macrolide of the first mucoadhesive film after removal of the film. The black bar shows the residual amount of macrolide of the second mucoadhesive film after removal of the film.

**Figure 7** shows the normalized concentration-time curves (tacrolimus blood concentrations at each measurement time point as a percentage of the maximum concentration) of a study with human subjects.

**Figure 8** shows the relatively small extent of inter-individual blood level fluctuations (CV) for tacrolimus in both groups over a 24-hour interval in a buccal application. Advantageously, buccal administration reduces blood level fluctuations compared to oral application.

In the following, reference is made to the examples, which are given to illustrate, not to limit the present invention.

EXAMPLES

**EXAMPLE 1**: CLINICALLY HUMAN PHASE I KINETIK STUDY (PROOF OF CONCEPT IN HUMANS)

The first human study with a mucoadhesive buccal film formulation of tacrolimus has been executed:

*Study rationale*

This first human pilot-study primarily aimed to investigate the PK of tacrolimus MBF in healthy male subjects (human proof-of-concept study). Additionally, the safety and tolerability of tacrolimus MBF was assessed and the residual amount of tacrolimus in MBF after use was determined.

To overcome PK issues associated with the currently available oral tacrolimus formulations, a novel tacrolimus formulation in the form of a mucoadhesive buccal film (MBF) was developed.

*Study objectives and endpoints*

| Objectives | Endpoints |
|---|---|
| **Primary** | |
| • To characterize the pharmacokinetics of tacrolimus administered by buccal route in whole blood | • $AUC_{0-4}$, $AUC_{4-8}$, $AUC_{0-24}$, $AUC_{0-tlast}$ and $AUC_{0-inf}$ (if feasible) of tacrolimus<br>• $C_{max}$ of tacrolimus<br>• $t_{max}$, $t_{1/2}$ and $\lambda_z$ of tacrolimus |
| **Secondary** | |
| • To investigate the safety and tolerability of tacrolimus administered by buccal route<br><br>• To determine the residual amount of tacrolimus in MBF after use | • Treatment-emergent adverse events (TEAEs) and severity of TEAEs; physical examination, vital sign, ECG and safety laboratory measurements; local tolerability, taste, sensation, mucoadherence assessment<br>• Residual amount of tacrolimus in the product after use |

*Methodology (design of study):*

This study was conducted in a single-center, open-label, non-randomized, multiple-dose, parallel-group design with 2 sequential treatment groups.

To overcome pharmacokinetic issues associated with the currently available oral tacrolimus formulations, a novel tacrolimus formulation in the form of a MBF was developed.

The aim of this study was to investigate the pharmacokinetics of tacrolimus MBF in healthy male subjects. Additionally, the safety and tolerability of tacrolimus MBF was assessed and the residual amount of tacrolimus in MBF after use was determined.

Treatments were planned as follows:

| Group | Treatment |
|---|---|
| Treatment Group 1 | Two single doses of 8.8 mg tacrolimus by sequential application of MBF-1: first right cheek, then left cheek |
| Treatment Group 2 | Two single doses of 12.0 mg tacrolimus by sequential application of MBF-2: first right cheek, then left cheek. |

| MBF-1 = tacrolimus mucoadhesive buccal film 8.8 mg<br>MBF-2 = tacrolimus mucoadhesive buccal film 12.0 mg |
|---|

The study was structured as follows:

- Screening visit: Within 21 to 2 days prior to the first administration of study treatment.

- Day -1: Admission of subjects to the study ward, assignment to study treatment.

- Day 1: Administration of 2 single doses of study treatment via buccal route. The time between the start of the 2 subsequent administrations in a particular subject was 4 h. Each MBF stayed attached to the mucosa of the cheek for 1 hour.

- Day 2: Discharge of subjects from study ward after completion of all protocol procedures and if no medical objection existed.

- Follow-up visit: At 1 to 3 days after last administration of study treatment.

Safety and tolerability parameters were collected during the entire study phase from screening to follow-up. Blood samples for PK analyses were collected up to 28 h after first administration of tacrolimus.

*Number of subjects (planned and analyzed):*

Two (2) sequential treatment groups receiving tacrolimus MBF were dosed. At least 10 healthy subjects were planned to be assigned to study treatment to have at least 5 evaluable subjects per treatment group completing the study.
Finally, 10 subjects were assigned to a treatment group and treated, i.e., 5 subjects in Treatment Group 1 and 5 subjects in Treatment Group 2.

*Diagnosis and main criteria for inclusion:*

Healthy male subjects (18 to 40 years, inclusive) with a body weight $\geq$ 65 kg and $\leq$ 85 kg and a body mass index (BMI) within the range of 18.0 to 30.0 kg/m$^2$ (inclusive) were eligible.

*Test products, dose and mode of administration, batch numbers:*

| Treatment | Test Product 1 | Test Product 2 |
|---|---|---|
| Compound Name | MBF-1 | MBF-2 |
| Active Substance | Tacrolimus | Tacrolimus |
| Dose Formulation | Mucoadhesive buccal film | Mucoadhesive buccal film |
| Unit Dose Strengths | 8.8 mg / 4.4 cm$^2$ | 12.0 mg / 6.0 cm$^2$ |
| Dosage Levels | 8.8 mg, twice daily | 12.0 mg, twice daily |
| Route of Administration | Buccal | Buccal |
| Batch number drug | 09166102E | 09166102E |

(continued)

| Treatment | Test Product 1 | Test Product 2 |
|---|---|---|
| substance | | |
| Batch number drug product (primary packaging) | 7009392 | 7009402 |
| Batch number label | D033G | D034G |

*Duration of treatment:*

Study duration was approximately 4 weeks per subject.

On Day 1, subjects were administered 2 single doses of study treatment. The time between the start of the 2 subsequent administrations in a particular subject was 4 h. Each MBF stayed attached to the mucosa of the cheek for 1 h.

*Statistical methods:*

Pharmacokinetics: No formal statistical hypotheses had been defined for this exploratory study. Appropriate summary tables and graphs were provided.

Safety: Safety data were evaluated descriptively.

*Summary and conclusions:*

Pharmacokinetics:

All subjects in Treatment Group 1 (T1) had whole blood concentration below LLOQ until 0.5 h after start of 1st MBF application and at 3 h after start of 1st MBF application (= 2 h after end of 1st MBF application) all subjects had whole blood concentration above LLOQ. In Treatment Group 2 (T2) all subjects had whole blood concentration below LLOQ until 0.25 h after start of 1st MBF application and at 1.5 h after start of 1st MBF application all subjects had whole blood concentration above LLOQ.

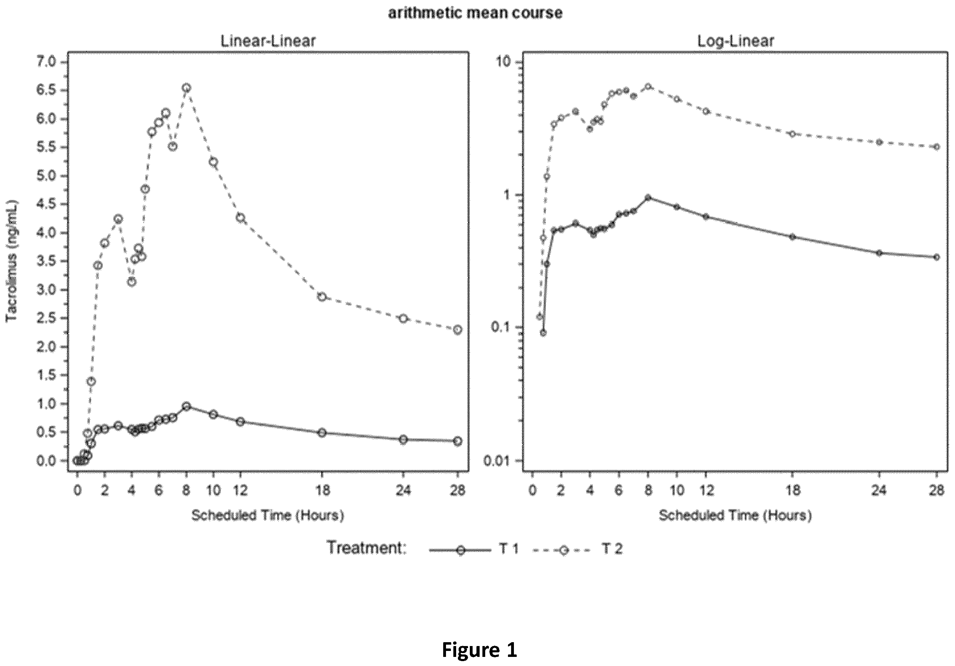

Mean whole blood concentration curves of both treatment groups reached a first peak at 3 h after start of 1st MBF application (= 2 h after end of 1st MBF application) and after a low decrease reached a second peak at 8 h after start of 1st MBF application (4 h after start of 2nd MBF application = 3 h after end of 2nd MBF application), showing a mean concentration of 0.9526 ng/mL (T1) and 6.548 ng/mL (T2). The results are presented in Fig. 1.

Geometric mean $AUC_{0-4}$ of tacrolimus was considerably higher in Treatment Group 2 (12.0 mg; 6.063 h*ng/mL) than in Treatment Group 1 (8.8 mg; 1.044 h*ng/mL). The same applies for geometric mean $AUC_{0-tlast}$ (T1: 13.594 h*ng/mL; T2: 56.021 h*ng/mL) and geometric mean $C_{max}$ (T1: 0.878 ng/mL; T2: 3.771 ng/mL).

Median $t_{max}$ was comparable between both treatment groups (T1: 8.083 h after start of 1st MBF application; = 3.083 h after end of 2nd MBF application; T2: 8.017 h after start of 1st MBF application; = 3.017 h after end of 2nd MBF application).

Geometric mean half-life and elimination rate were also comparable (t1/2: T1: 14.475 h and T2: 14.926 h; $\lambda z$: T1: 0.04789 1/h and T2: 0.04644 1/h).

Particularly in Treatment Group 2, subjects showed individual differences concerning pharmacokinetic parameters. Subject 015 hat the highest $C_{max}$ value (17.5 ng/ml) which was 24.9-fold higher than the lowest measured $C_{max}$ (Subject 012; 0.703 ng/ml). Subject 015 showed also the highest $AUC_{0-tlast}$ (224.76 h*ng/ml) which was 19.5-fold higher than the lowest $AUC_{0-tlast}$ (Subject 012; 11.51 h*ng/ml).

*Summary of the main PKparameters for tacrolimus (PK Set, N = 10)*

| | | Treatment Group 1 | Treatment Group 2 |
|---|---|---|---|
| AUC0-4 (h*ng/mL) | Geom. Mean (CV%) | 1.044 (159.1) | 6.063 (268.0) |
| AUC4-8 (h*ng/mL) | Geom. Mean (CV%) | 2.420 (56.0) | 11.022 (283.2) |
| AUC0-24 (h*ng/mL) | Geom. Mean (CV%) | 12.294 (54.5) | 50.984 (240.0) |

(continued)

| | | Treatment Group 1 | Treatment Group 2 |
|---|---|---|---|
| AUC0-tlast (h*ng/mL) | Geom. Mean (CV%) | 13.594 (53.9) | 56.021 (243.2) |
| Cmax (ng/mL) | Geom. Mean (CV%) | 0.8780 (49.5) | 3.771 (305.4) |
| tmax (h) | Median | 8.083 | 8.017 |
| | Min-Max | 8.07 - 9.98 | 3.00 - 10.00 |
| t1/2 (h) | Geom. Mean (CV%) | 14.475 (22.9) | 14.926 (10.7) |
| Lambda z (1/h) | Geom. Mean (CV%) | 0.04789 (22.9) | 0.04644 (10.7) |

Treatment Group 1: two doses of tacrolimus MBF 8.8 mg
Treatment Group 2: two doses of tacrolimus MBF 12.0 mg
CV = coefficient of variation; Geom. = geometric; Max = maximum; Min = minimum, N = number of subjects in the population under consideration.

In total, high amounts of tacrolimus remained in the MBFs. The mean percentage of residual amount of tacrolimus in the MBF was after each dosing higher in Treatment Group 1 (1st application: 91.076%; 2nd application: 85.650%) than in Treatment Group 2 (1st application: 78.214%; 2nd application: 74.296%).

*First Human Kinetic Data of the Tacrolimus - MBF*

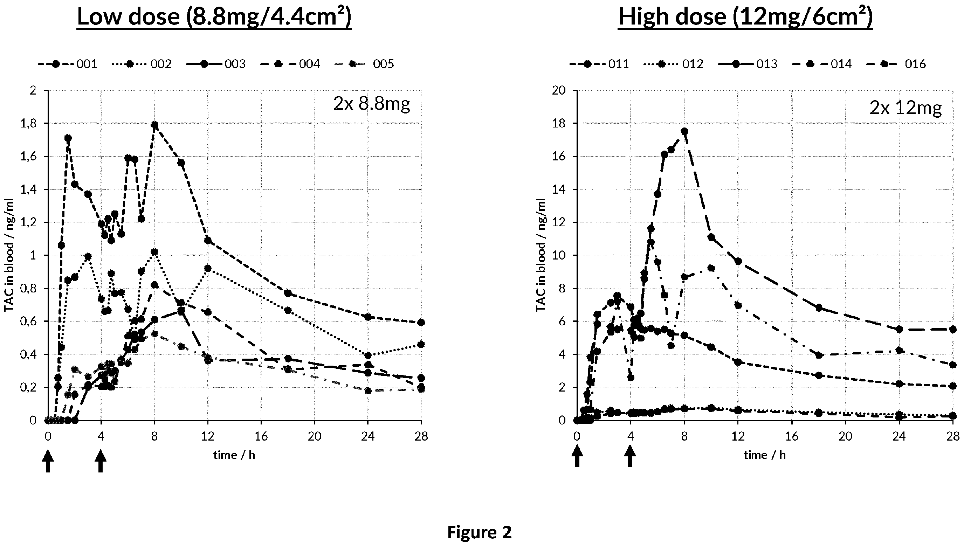

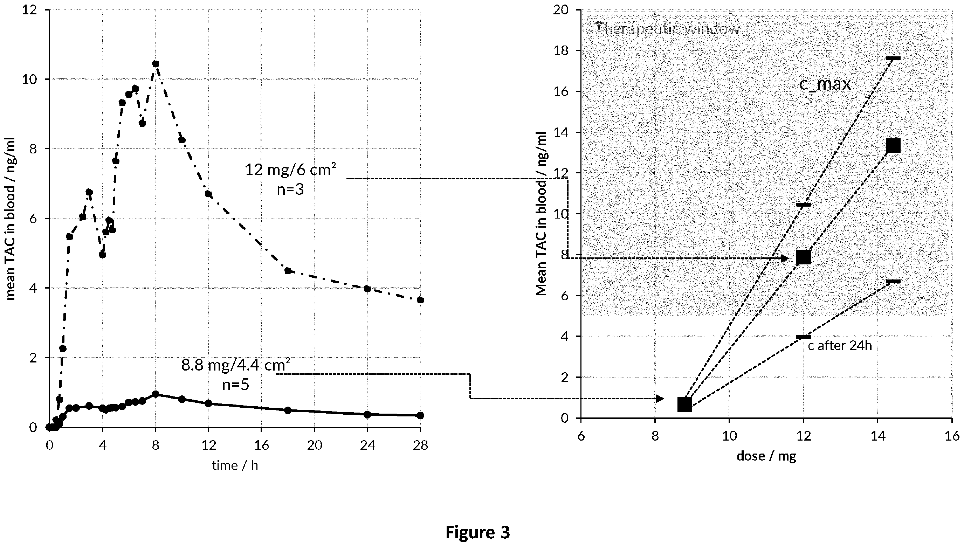

Individual tacrolimus blood levels in 10 healthy volunteers (5 subjects per group), bid application, second dose after 4 hours are shown in Figure 2.
Tacrolimus mean blood level profiles in 10 healthy volunteers (5 subjects per group), bid application, second dose after 4 hours are shown in Figure 3.

*Conclusions*

- The results of the first human kinetic indicate that systemic exposure was achieved after buccal administration of the unidirectional releasing MBF formulation, which provides proof-of-concept in humans.
- In a clinical setting, the dosing of tacrolimus is usually adjusted according to body weight, while in this pilot PK study a fixed nominal dose was administered to all subjects of a particular dose group. This likely contributed to the observed variability of tacrolimus blood levels.

**EXAMPLE 2:** DOSING REGIMEN FOR BUCCAL APPLICATION

The current recommendations for *oral* tacrolimus standard therapy (prograf caps.) for adult patients with kidney, liver, or heart transplants are:

| Kidney | 0,2 mg/kg bw/day, devided in two doses, administered very 12 hours |
|---|---|
| Liver | 0,10-0,15 mg/kg bw/day, devided in two doses, administered every 12 hours |
| Heart | 0,075 mg/kg bw / day devided in two doses, administered every 12 hours |

Tacrolimus drug monitoring (TDM) is recommended for all patients receiving Prograf.
Monitoring of tacrolimus blood concentrations in conjunction with other laboratory and clinical parameters is considered an essential aid to patient management for the evaluation of rejection, toxicity, dose adjustments, and compliance.
The novel buccal application route will introduce a new dosing regimen for the daily tacrolimus therapy to cover a 24h dosing interval.
Based on the kinetics data from the preclinical animal studies, a twice-daily application (bid) with 4-h time interval between the first and the second dose was chosen for the human kinetics study.
The blood level profiles of the high-dose group (n = 5 healthy subjects), loaded with 12 mg tacrolimus per film unit, demonstrate, that clinically relevant blood levels are achieved with a twice-daily application system and a 4-h time interval between the first and the second dose.

In order, to validate this specific asymmetric dosing regimen for buccal application, a further animal study was performed under GLP conditions, using 3 different application variants:

*Study design (GLP-study):*

- Intra-individual 3-way cross-over
- 3-group-comparison
- bid application (5 naive mini pigs per group, GLP)

| Sex # Animal | Parameter | Session 1 | Session 2 | Session 3 |
|---|---|---|---|---|
| | Formulation | **Mucoadhesive buccal film** | | |
| | Batch number | 81208822 | | |
| F#1 - #5 | Dose per film | 14.4 mg | | |
| | Film area | 5 cm$^2$ | | |
| | Residence time | 60 min | | |
| | Treatment | twice a day - 3 h between | twice a day - 4 h between | twice a day - 5 h between |
| | Application site | unilateral | unilateral | unilateral |

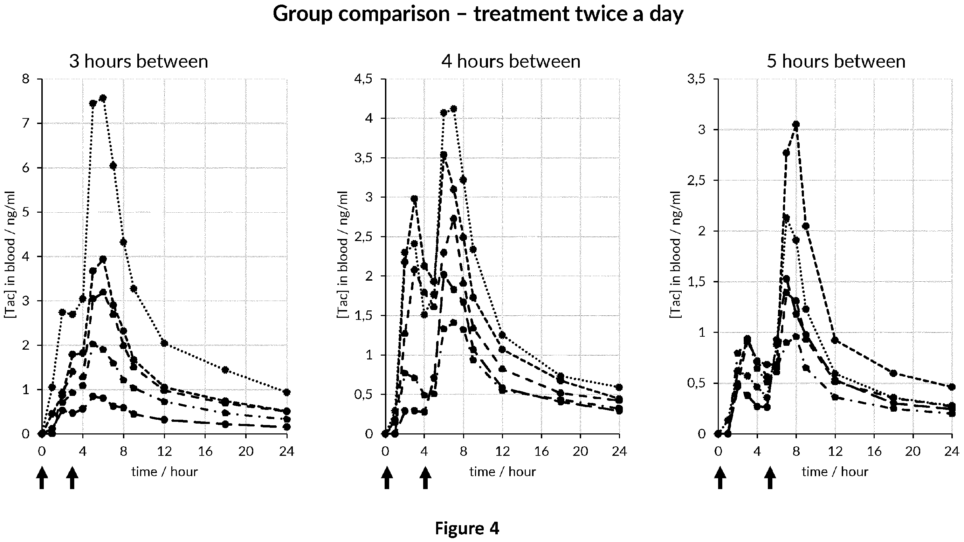

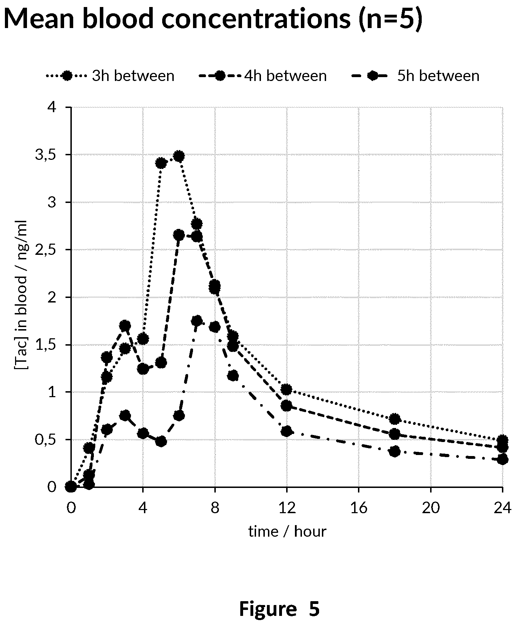

Results: the individual PK-profiles within the different groups are shown in Figure 4. As shown in Figure 5, the plot of the mean curves proves that the highest blood levels are obtained with a bid-dosing-regimen, when the second dose unit is administered after 3h. The tested asymmetric dosing schemes are highly advantageous. Particularly, the asymmetric 3h-bid application system shows excellent results with respect to the achieved blood concentrations.

**EXAMPLE 3:** DOSE REDUCTION BASED ON INCREASED BIOAVAILABILITY

The daily oral tacrolimus standard dose (Prograf) for a SOT-patient is calculated according to the formula:

$$\text{mg Tacrolimus} / \text{kg bw} / \text{day}$$

The starting dose of oral tacrolimus for a 70kg patient, who has received a kidney, thus results in a calculated daily dose of:

$$0.2\text{mg} \times 70\text{kg} / \text{day} = 14 \text{ mg daily dose}$$

Split into two single doses (morning does / evening dose).

This dosage guideline is part of the international registration documentation of oral Prograf capsules, the current standard in oral tacrolimus therapy.

For the buccal therapy system, the situation is different:

In the human study presented here, buccal films were used at two different dose levels:

- Low dose: loading with 8.8mg tacrolimus = 17.6 mg (bid).
- High dose: loading with 12 mg tacrolimus = 24 mg (bid).

The residence time of the films on the buccal mucosa after application is 60 min; thereafter, the film is actively removed from the oral cavity.

Only the biologically available portion of active ingredient that penetrates from the film matrix into the mucosal tissue within 60 min and is subsequently released into the systemic circulation by passive diffusion is relevant for achieving therapeutic blood levels.

This resorption process typically occurs in three steps:

Step 1: drug release into mucosal tissue
Step 2: building API depot in mucosa
Step 3: diffusion into blood circulation

The residual tacrolimus content in the used films after removal from the cheek was analyzed to determine how much drug penetrated into the mucosal tissue building the depot for diffusion. Figure 6 shows the residual drug levels in the used films of both dose groups.

*Results:*

1. Remaining Tacrolimus post application (average of films)

- 8.8mg film: 88.4% $\pm$ 5.6% (low-dose group)

- 12mg film: 81.8% $\pm$ 8.0% (high-dose-group)

More than *80%* of applied Tacrolimus remain in the film.

2. Advantageously, only a lower buccal dose of Tacrolimus is necessary to reach therapeutic blood levels:
Dosing example (patient, bw = 80kg):

$$\text{Standard oral dose} = 16 \text{ mg Tacrolimus / day}$$

$$\textit{buccal} \text{ application} = 3,2 \text{ mg Tacrolimus / day}$$

These data confirm that, advantageously, the bioavailability of the drug absorbed into the buccal mucosa is much higher compared to oral application, because after buccal application only about 20% of the oral dose is necessary to achieve therapeutic levels.

Without wishing to be bound by any theory, the inventors believe that these effects can be scientifically explained by the fact that the low bioavailability of Tacrolimus (BCS Class II), the pronounced first-pass-effect, as well as the pre-systemic degradation of tacrolimus after oral administration are not relevant in buccal application.

Advantageously, since it is possible to reduce the drug load for the patient by approx. 80%, the side effect profile of tacrolimus therapy can also be positively changed.

**EXAMPLE 4:** Blood level variability

One of the most difficult aspects of therapy management with tacrolimus is the significant intra- and interpatient variability of the concentration-time-profile after oral administration of tacrolimus.

The reasons for this variability are well known and sufficiently described in the international literature: (1) poor water solubility and thus low bioavailability of the drug with high variability (4-90%), (2) first-pass effect, (3) pre-systemic degradation, (4) food effect.

All these effects are not relevant in the buccal delivery system due to better bioavailability and direct access to systemic circulation.

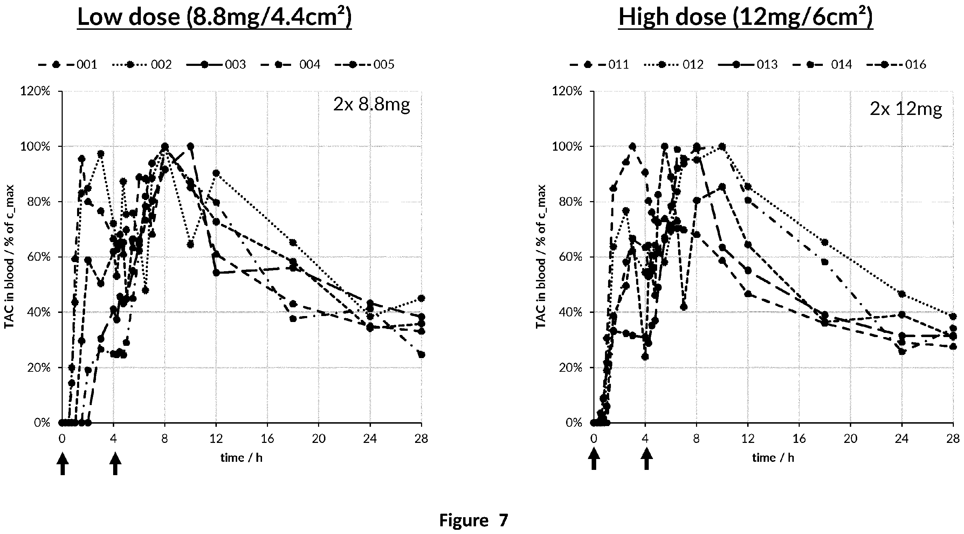

This is also shown by the data of the present human study (Figure 7). The diagrams presented in Figure 7 show the normalized concentration-time curves (tacrolimus blood concentrations at each measurement time point as a percentage of the maximum concentration) of both dose groups.

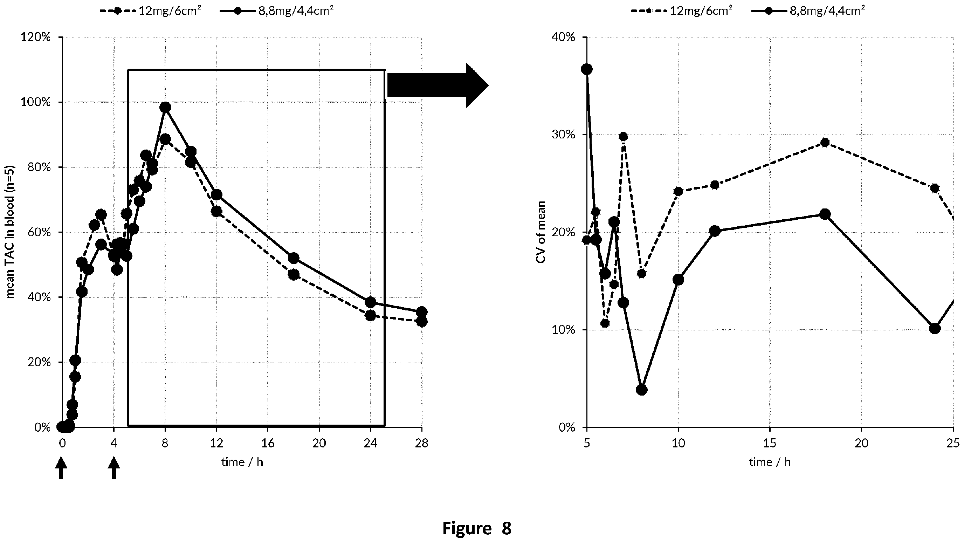

Comparing the mean values of the normalized concentration-time profiles of both groups with each other, the relatively small extent of inter-individual blood level fluctuations (CV) for tacrolimus in both groups over a 24-hour interval can be illustrated (Figure 8). Compared to the internationally described blood level fluctuations of tacrolimus after oral application with a variation range of more than 80%, the blood level fluctuations after buccal application presented here with a variation range of 25 - 30% are to be classified as low. Thus, advantageously, the herein described buccal administration reduces blood level fluctuations compared to oral application.

Without wishing to be bound by any theory, the inventors believe that one reason for the smaller range of variation of the blood level profiles after buccal administration compared to oral administration of tacrolimus is the better bioavailability of the active substance after buccal administration (see Example 2).

The primary goal of patient-individualized dose titration with tacrolimus is to adjust the blood level profile over a 24h therapy interval in such a way that it remains on average over 24h (Mean Residence Time (MRT)) in the therapeutic window between 5 - 20ng/ml whole blood. Of particular clinical importance in this context are the blood level fluctuations of tacrolimus - minimum levels - values ($C_{trough}$) at the end of the dosing interval after 24 h, i.e. the pre-dose levels before the next administration.

There is a direct correlation between time staying in the therapeutic window and the clinically effectiveness in the first year after transplantation. Consequently, the dosing regimen (bid) and the individual daily dose are preferably designed in a way that at the end of a 24h dosing interval, tacrolimus blood levels are still in the therapeutic window.

In the present human kinetic study, it was shown in both dosing groups that with the chosen bid dosing regimen, $C_{trough}$ blood levels after 24hours are representing 30-40% of Cmax-values. This high $C_{trough}$ blood level values after 24h are important for a safe and clinically effective therapy management with tacrolimus.

In addition, the low inter-individual blood level variations compared to oral therapy underline the additional benefit of the herein presented innovative buccal therapy system.

**EXAMPLE 5:** EXEMPLARY COMPOSITIONS OF THE MUCOADHESIVE FILM

Exemplary compositions of the applied tacrolimus mucoadhesive buccal films (8.8 mg/4.4 cm$^2$), by layer, were as follows:

| Components | Theoretical quantity | | | Reference to standard | Function |
|---|---|---|---|---|---|
| | [mg/cm$^2$] | [mg/ unit] | [%] | | |
| **backing layer** | | | | | |
| ethyl cellulose [2] | 3.0 | 13.2 | 59.90 | Ph. Eur. | lipophilic film former |
| polyvinyl acetate / povidone | 1.5 | 6.6 | 30.00 | in-house | lipophilic film former |
| triacetin | 0.5 | 2.2 | 10.00 | Ph. Eur. | plasticizer |
| Brilliant Blue | 0.01* | 0.02 | 0.10 | in-house | coloring agent |
| ethanol anhydrous [5] | q.s. | q.s. | q.s. | Ph. Eur. | process solvent |
| acetone [5] | q.s. | q.s. | q.s. | Ph. Eur. | process solvent |
| **adhesive layer** | | | | | |
| ethyl cellulose [2] | 0.3 | 1.5 | 5.00 | Ph. Eur. | lipophilic film former |
| polyvinyl acetate / povidone | 5.6 | 24.6 | 80.00 | in-house | lipophilic film former |
| glycerol | 1.0 | 4.6 | 15.00 | Ph. Eur. | plasticizer |
| ethanol anhydrous [5] | q.s. | q.s. | q.s. | Ph. Eur. | process solvent |
| acetone [5] | q.s. | q.s. | q.s. | Ph. Eur. | process solvent |
| **mucoadhesive layer** | | | | | |
| Tacrolimus [1] | 2.0 | 8.8 | 13.33 | Ph. Eur. | API |
| ethyl cellulose [2] | 4.5 | 19.7 | 29.84 | Ph. Eur. | lipophilic film former |
| hydroxypropyl cellulose EF [3] | 5.2 | 23.0 | 34.92 | Ph. Eur. | amphiphilic film former |
| hydroxypropyl cellulose GF [4] | 1.2 | 5.3 | 8.05 | Ph. Eur. | amphiphilic film former |
| copovidone | 0.4 | 1.7 | 2.50 | in-house | hydrophilic film former / solubilizer |
| glycerol | 0.7 | 2.9 | 4.44 | Ph. Eur. | plasticizer |

(continued)

| mucoadhesive layer | | | | | |
|---|---|---|---|---|---|
| calcium/sodium PVM/MA-copolymer | 0.5 | 2.3 | 3.54 | in-house | mucoadhesive |
| titanium dioxide | 0.5 | 2.2 | 3.38 | Ph. Eur. | whitening agent |
| ethanol anhydrous [5] | q.s. | q.s. | q.s. | Ph. Eur. | process solvent |

[1] weighed as Tacrolimus $H_2O$: 9.0 mg Tacrolimus $H_2O$ correspond to 8.8 mg Tacrolimus

[2] e.g. EC N50NF

[3] e.g. Klucel EF

[4] e.g. Klucel GF 5 partially removed during drying process, please refer to 2.1.P.5.1 * rounded value, actually 0.005

Exemplary compositions of the applied tacrolimus mucoadhesive buccal films (12.0 mg/6.0 $cm^2$), by layer, were as follows:

| Components | Theoretical quantity | | | Reference to standard | Function |
|---|---|---|---|---|---|
| | [mg/cm²] | [mg/unit] | [%] | | |
| **backing layer** | | | | | |
| ethyl cellulose [2] | 3.0 | 18.0 | 59.90 | Ph. Eur. | lipophilic film former |
| polyvinyl acetate / povidone | 1.5 | 9.0 | 30.00 | in-house | lipophilic film former |
| triacetin | 0.5 | 3.0 | 10.00 | Ph. Eur. | plasticizer |
| Brilliant Blue | 0.01* | 0.03 | 0.10 | in-house | coloring agent |
| ethanol anhydrous [5] | q.s. | q.s. | q.s. | Ph. Eur. | process solvent |
| acetone [5] | q.s. | q.s. | q.s. | Ph. Eur. | process solvent |
| **adhesive layer** | | | | | |
| ethyl cellulose [2] | 0.35 | 2.1 | 5.00 | Ph. Eur. | lipophilic film former |
| polyvinyl acetate / povidone | 5.6 | 33.6 | 80.00 | in-house | lipophilic film former |
| glycerol | 1.1 | 6.3 | 15.00 | Ph. Eur. | plasticizer |
| ethanol anhydrous [5] | q.s. | q.s. | q.s. | Ph. Eur. | process solvent |
| acetone [5] | q.s. | q.s. | q.s. | Ph. Eur. | process solvent |
| **mucoadhesive layer** | | | | | |
| Tacrolimus [1] | 2.0 | 12.0 | 13.33 | Ph. Eur. | API |
| ethyl cellulose [2] | 4.5 | 26.8 | 29.84 | Ph. Eur. | lipophilic film former |
| hydroxypropyl cellulose EF [3] | 5.2 | 31.4 | 34.92 | Ph. Eur. | amphiphilic film former |
| hydroxypropyl cellulose GF [4] | 1.2 | 7.2 | 8.05 | Ph. Eur. | amphiphilic film former |
| copovidone | 0.4 | 2.3 | 2.50 | in-house | hydrophilic film former / solubilizer |
| glycerol | 0.7 | 4.0 | 4.44 | Ph. Eur. | plasticizer |
| calcium/sodium PVM/MA-copolymer | 0.5 | 3.2 | 3.54 | in-house | mucoadhesive |

(continued)

| mucoadhesive layer | | | | | |
|---|---|---|---|---|---|
| titanium dioxide | 0.5 | 3.0 | 3.38 | Ph. Eur. | whitening agent |
| ethanol anhydrous [5] | q.s. | q.s. | q.s. | Ph. Eur. | process solvent |

[1] weighed as Tacrolimus $H_2O$: 12.3 mg Tacrolimus $H_2O$ correspond to 12.0 mg Tacrolimus

[2] e.g. EC N50NF

[3] e.g. Klucel EF

[4] e.g. Klucel GF 5 partially removed during drying process, please refer to 2.1.P.5.1 * rounded value, actually 0.005

The features of the present invention disclosed in the specification, the claims, and/or in the accompanying figures may, both separately and in any combination thereof, be material for realizing the invention in various forms thereof.

**Claims**

1. A macrolide for use in a method of preventing or treating an immunological disease or disorder, preferably a transplant rejection, comprising administering said macrolide at least twice daily,

   wherein said administering at least twice daily comprises administering a first dose at a first time point and a second dose at a second time point, wherein said second time point is at a time point in a range of from about 1 h to about 7 h, preferably of from about 2 h to about 5 h, more preferably of from about 2.5 h to about 3.5 h, even more preferably about 3 h, after said first time point;
   wherein, preferably, said macrolide is administered by buccal administration.

2. The macrolide for use according to claim 1, wherein said macrolide is administered in the form of a mucoadhesive layer, preferably mucoadhesive buccal layer;
   wherein, preferably, said administering comprises attaching said mucoadhesive layer to a body cavity of a patient, preferably to a buccal cavity of a patient and/or to a mucosa of a patient.

3. The macrolide for use according to claim 1 or 2, wherein said administering comprises administering each of said first dose and said second dose, preferably each in the form of a mucoadhesive layer, for a duration in a range of from about 1 min to about 7 h, preferably of from about 10 min to about 3 h, more preferably of from about 20 min to about 2 h, even more preferably of from about 30 min to about 90 min, even more preferably of about 60 min.

4. The macrolide for use according to any one of the foregoing claims, wherein said administering comprises administering said first dose for a duration in a range of from about 30 min to about 90 min, preferably for about 60 min; and administering said second dose for a duration in a range of from about 30 min to about 90 min, preferably for about 60 min, at a time point in a range of from about 2.5 h to about 3.5 h, preferably about 3 h, after said first time point.

5. The macrolide for use according to any one of the foregoing claims, wherein said first dose and said second dose, preferably prior to said administration thereof, each comprise said macrolide in an amount in a range of from about 0.001 mg to about 250 mg, preferably in an amount in a range of from about 0.01 mg to about 50 mg, more preferably in a range of from about 0.05 mg to about 25 mg, even more preferably in a range of from about 0.1 mg to about 15 mg, even more preferably in a range of from about 0.5 mg to about 13 mg, optionally in a range of from about 8 mg to about 12 mg; and/or
   wherein said macrolide is administered by buccal administration, and wherein said administering said macrolide at least twice daily comprises administering a total daily amount of macrolide which is about ≤ 40%, preferably about ≤ 30%, more preferably about ≤ 25%, even more preferably about ≤ 20%, of a total daily amount administered by an oral administration.

6. The macrolide for use according to any one of claims 2 to 5, wherein said macrolide is present in said mucoadhesive layer, prior to said administration, in an amount in the range of from approximately 0.01 mg to approximately 10 mg, with reference to 1 cm$^2$ of said mucoadhesive layer, preferably in an amount in the range of from approximately 0.1 mg to approximately 5 mg, with reference to 1 cm$^2$ of said mucoadhesive layer, more preferably in the range of from approximately 1.5 mg to approximately 3.5 mg, with reference to 1 cm$^2$ of said mucoadhesive layer, even more preferably in the range of from approximately 1.8 mg to approximately 2.5 mg, with reference to 1 cm$^2$ of said mucoadhesive layer.

7. The macrolide for use according to any one of the foregoing claims, wherein said macrolide is administered in the form of a mucoadhesive layer comprising

- a mucoadhesive polymer, preferably a polymer selected from a) amphiphilic polymers and hydrophilic polymers, b) poly(methacrylates), c) crosslinked polyacrylic acid polymers, d) copolymers of methyl vinyl ether and maleic anhydride, e) polymers comprising polyvinyl acetate and/or polyvinylpyrrolidone, and combinations thereof;

wherein, preferably,
the amphiphilic polymers are selected from polyvinyl caprolactam-polyvinyl acetate-polyethylene glycol graft copolymers, polyoxyl castor oils and D-α-tocopherol-polyethylenglycol-succinate (TPGS), and/or the poly(methacrylates) are selected from neutral protonizable poly(methacrylates) and cationic poly(methacrylates), preferably selected from copolymers of dialkylaminoethyl methacrylates and methacrylic acid ester(s), and copolymers of trialkylammonioethyl methacrylates and methacrylic acid ester(s), in particular copolymers of dimethylaminoethyl methacrylates and methacrylic acid esters, and copolymers of trimethylammonioethyl methacrylates and methacrylic acid esters;

and wherein, optionally, the mucoadhesive layer further comprises:

- a cellulose derivative, preferably selected from hydroxypropyl cellulose (HPC), carboxymethyl cellulose (CMC), hydroxypropyl methylcellulose (HPMC), hydroxyethyl cellulose (HEC), methyl cellulose (MC), hydroxyethyl methyl cellulose (HEMC), ethyl cellulose (EC), and a combination thereof;
- a plasticizer; preferably selected from glycerol, polyethylene glycol, e.g. low molecular weight polyethylene glycol such as PEG 200 or PEG 400, propylene glycol, sorbitol, triacetin, and tributylcitrate; more preferably glycerol; and/or
- a colorant, preferably TiO$_2$;

wherein, optionally, said macrolide is present in said layer in the form of micronized macrolide, preferably micronized crystalline macrolide, and/or in a molecularly dissolved non-nanoparticulate form.

8. The macrolide for use according to any one of the foregoing claims, wherein said administering at least twice daily provides a first peak of a macrolide whole blood concentration at a time point in a range of from about 2 h to about 4 h, preferably of from about 2.5 h to about 3.5 h, more preferably about 3 h, after said first time point, and a second peak of said macrolide whole blood concentration at a time point in a range of from about 7 h to about 9 h, preferably of from about 7.5 h to about 8.5 h, more preferably about 8 h, after said first time point;
wherein, optionally, said administering at least twice daily provides said second peak of said macrolide whole blood concentration at a time point in a range of from about 3 h to about 5 h, preferably of from about 3.5 h to about 4.5 h, more preferably about 4 h, after said second time point.

9. The macrolide for use according to any one of the foregoing claims, wherein said administering at least twice daily provides a whole blood concentration of said macrolide in a range of from about 0.01 ng/mL to about 50 ng/mL, preferably in a range of from about 3 ng/mL to about 20 ng/mL, more preferably in a range of from about 5 ng/mL to about 20 ng/mL, for a duration of about 24 hours.

10. The macrolide for use according to any one of the foregoing claims, wherein said administering at least twice daily provides a coefficient of variation below 40%, preferably below 35%, more preferably below 32%, even more preferably 30% or less, such as in a range of from 20% to 30% or in a range of from 25% to 30%.

11. The macrolide for use according to any one of the foregoing claims, wherein said administering at least twice daily provides

- a $t_{max}$ in a range of from about 3 h to about 10 h after said first time point, preferably in a range of from about 7 h to about 9 h after said first time point, more preferably in a range of from about 7.5 h to about 8.5 h after said first time point; and/or

- a $t_{max}$ in a range of from about 1 h to about 6 h after said second time point, preferably in a range of from about 2 h to about 5 h after said second time point, more preferably in a range of from about 3.5 h to about 4.5 h after said second time point, e.g. about 4 h after said second time point.

12. The macrolide for use according to any one of the foregoing claims, wherein said administering at least twice daily provides

- a $C_{max}$ in a range of from about 0.05 ng/mL to about 50 ng/mL, preferably in a range of from about 0.1 ng/mL to about 30 ng/mL, more preferably in a range of from about 0.7 ng/mL to about 20 ng/mL, even more preferably in a range of from about 5.5 ng/mL to about 20 ng/mL; and/or
- a $C_{trough}$ in a range of from about 0.01 ng/mL to about 18 ng/mL, preferably in a range of from about 0.5 ng/mL to about 16 ng/mL, more preferably in a range of from about 3 ng/mL to about 15 ng/mL, such as in a range of from about 3 ng/mL to about 7 ng/mL or in a range of from about 5 ng/mL to about 7 ng/mL.

13. The macrolide for use according to any one of the foregoing claims, wherein said administering at least twice daily is performed for a duration of at least 2 days, preferably at least 1 week, more preferably at least 1 month, even more preferably at least 1 year; wherein, optionally, if said macrolide is for use in preventing or treating a transplant rejection, said administering at least twice daily is performed for a lifetime of a patient.

14. The macrolide for use according to any one of the foregoing claims, wherein said macrolide is administered to a human patient; wherein, preferably, said patient is not a fasting patient.

15. The macrolide for use according to any one of the foregoing claims, wherein the macrolide is selected from macrolide immune suppressants, macrolide antibiotics, and macrolide antifungals;

wherein, preferably,

- the macrolide immune suppressant is selected from tacrolimus, sirolimus, everolimus, and pimecrolimus;
- the macrolide antibiotic is selected from erythromycin, clarithromycin, azithromycin, roxithromycin, josamycin, spiramycin, telithromycin, tylosin, and fidaxomicin; and/or
- the macrolide antifungal is selected from polyenes, in particular nystatin, natamycin, and amphotericin B,

wherein, more preferably, said macrolide is tacrolimus or sirolimus, even more preferably tacrolimus.

arithmetic mean course

Figure 1

EP 4 410 287 A1

Figure 2

**Figure 3**

# Group comparison – treatment twice a day

**Figure 4**

Mean blood concentrations (n=5)

Figure 5

EP 4 410 287 A1

Figure 6

EP 4 410 287 A1

**Low dose (8.8mg/4.4cm²)**

**High dose (12mg/6cm²)**

Figure 7

Figure 8

EP 4 410 287 A1

Europäisches
Patentamt

European
Patent Office

Office européen
des brevets

# EUROPEAN SEARCH REPORT

Application Number

EP 23 15 5166

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/263463 A1 (NUCLEUS MEDICAL GMBH [DE]) 22 December 2022 (2022-12-22) | 1-15 | INV.<br>A61K31/436<br>A61K9/00<br>A61P37/00 |
| Y | * claims 1-3, 5, 7, 8, 15; examples 10, 11; tables 15, 17 * | 13 | |
| Y | WO 2020/115140 A1 (LEON NANODRUGS GMBH [DE]) 11 June 2020 (2020-06-11)<br>* claims 12, 15, 19 * | 13 | |
| Y | Anonymous: "Evaluation of Mucoadhesive Tacrolimus Patch on Caspase-3 Inducing Apoptosis in Oral Lichen Planus – Tabular View – ClinicalTrials.gov",<br>,<br>18 November 2021 (2021-11-18),<br>XP093059777,<br>Retrieved from the Internet:<br>URL:https://classic.clinicaltrials.gov/ct2/show/record/NCT05139667<br>[retrieved on 2023-06-30]<br>* the whole document * | 13 | |

TECHNICAL FIELDS
SEARCHED (IPC)

A61K
A61P

The present search report has been drawn up for all claims

| Place of search | Date of completion of the search | Examiner |
|---|---|---|
| Munich | 3 July 2023 | Lo Giudice, M |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.........................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 23 15 5166

This annex lists the patent family members relating to the patent documents cited in the above-mentioned European search report.
The members are as contained in the European Patent Office EDP file on
The European Patent Office is in no way liable for these particulars which are merely given for the purpose of information.

03-07-2023

| Patent document cited in search report | Publication date | Patent family member(s) | Publication date |
|---|---|---|---|
| WO 2022263463 A1 | 22-12-2022 | EP 4104816 A1 | 21-12-2022 |
| | | US 2022395453 A1 | 15-12-2022 |
| | | WO 2022263463 A1 | 22-12-2022 |
| WO 2020115140 A1 | 11-06-2020 | AU 2019392718 A1 | 10-06-2021 |
| | | CA 3063417 A1 | 04-06-2020 |
| | | CN 113242732 A | 10-08-2021 |
| | | DK 3662894 T3 | 20-09-2021 |
| | | EP 3662894 A1 | 10-06-2020 |
| | | EP 3846783 A1 | 14-07-2021 |
| | | ES 2894112 T3 | 11-02-2022 |
| | | HU E056067 T2 | 28-01-2022 |
| | | IL 283458 A | 29-07-2021 |
| | | JP 2022513697 A | 09-02-2022 |
| | | KR 20210100142 A | 13-08-2021 |
| | | PL 3662894 T3 | 10-01-2022 |
| | | SG 11202105778P A | 29-06-2021 |
| | | SI 3662894 T1 | 30-11-2021 |
| | | US 2020170934 A1 | 04-06-2020 |
| | | US 2022079872 A1 | 17-03-2022 |
| | | WO 2020115140 A1 | 11-06-2020 |

EPO FORM P0459

For more details about this annex : see Official Journal of the European Patent Office, No. 12/82